# FASCICULE DE BREVET EUROPEEN

(11) **EP 1 502 582 B1**
(45) Date de publication et mention de la délivrance du brevet: **26.11.2008**
(21) Numéro de dépôt: 04291955.5
(22) Date de dépôt: 30.07.2004
(51) Int. Cl.: A61K 8/29, A61K 8/86, A61K 8/87, A61K 8/73, A61K 8/81, A61Q 17/04

(54) **Emulsions photoprotectrices huile-dans-eau contenant des agents tensioactifs géminés et des polymères associatifs**
Öl-in-Wasser-Sonneschutzemulsionen enthaltend Gemini Tenside und assoziative Polymere
O/W sunscreen emulsions containing gemini surfactants and associative polymers

(30) Priorité: 01.08.2003 FR 0309540
(43) Date de publication de la demande: 02.02.2005
(73) Titulaire: L'ORÉAL, 75008 Paris (FR)
(72) Inventeur: Candau, Didier, 91570 Bievres (FR); Gombert, Christèle, 60260 Lamorlaye (FR)
(74) Mandataire: Casalonga, Axel

(56) Documents cités:
- EP-A- 1 084 695
- EP-A- 1 093 796
- EP-A- 1 174 450
- WO-A-03/024412

## Description

La présente invention concerne des compositions photoprotectrices destinées à la protection de la peau et/ou des cheveux contre le rayonnement UV, contenant des nanopigments à base d'oxydes métalliques dans une phase liquide huile-dans-eau émulsionnée par un agent tensioactif géminé en présence d'un polymère associatif non ionique comportant une chaîne grasse en C₈₋₄₀, ainsi qu'un procédé de préparation de telles compositions.

De nombreuses compositions cosmétiques destinées à la photoprotection de la peau ont été proposées à ce jour. Ces compositions contiennent généralement, dans un support liquide émulsionné (émulsion huile-dans-eau), une ou plusieurs molécules organiques, capables d'absorber le rayonnement ultraviolet, solubles dans la phase huileuse et/ou aqueuse. L'utilisation de nanopigments minéraux dans de telles compositions solaires est de plus en plus fréquente car ces particules, invisibles à l'oeil nu grâce à leur petite taille, permettent d'augmenter l'indice de protection des compositions les contenant.

Parmi ces produits solaires connus, les émulsions huile-dans-eau fluides sont, d'une manière générale, plus appréciées par les consommateurs que les émulsions plus épaisses car elles ont un toucher plus agréable et permettent une application plus facile du produit.

L'un des inconvénients majeurs de ces compositions anti-solaires fluides contenant des nanopigments minéraux, réside dans la difficulté de concilier une bonne stabilité du produit et une protection efficace. En effet, pour obtenir des émulsions de faible viscosité, c'est-à-dire des émulsions dans lesquelles les gouttelettes de la phase dispersée sont de très petite taille, il est généralement nécessaire d'utiliser des moyens d'agitation extrêmement puissants tels que des homogénéisateurs à haute pression. La fabrication d'émulsions fluides avec de tels équipements est très coûteuse et ne permet pas l'introduction de nanopigments pendant la phase d'émulsification. En effet, les particules peuvent colmater les fines buses de ces appareils et peuvent, lorsque, comme le dioxyde de titane, elles ont une dureté importante, avoir un effet fortement abrasif entraînant la détérioration de l'homogénéisateur.

Une approche pour remédier à ce problème a consisté à incorporer les nanopigments non pas avant ou pendant l'étape d'émulsification mais après celle-ci. Pour cela, il a été proposé de mélanger des émulsions concentrées ne contenant qu'une partie de la phase aqueuse finale, avec une dispersion aqueuse du nanopigment. Ces dispersions aqueuses doivent être stabilisées avec des agents dispersants et l'on introduit ainsi dans l'émulsion des quantités relativement importantes de molécules ayant une activité de surface et qui sont susceptibles de perturber le film d'agents tensioactifs à l'interface huile/eau. La stabilité de telles émulsions est en général limitée dans le temps et le produit final ne présente plus alors les qualités organoleptiques et l'efficacité de protection indispensables à sa commercialisation.

Une autre possibilité consiste à incorporer les nanopigments non pas sous forme de dispersion aqueuse mais sous forme pulvérulente. L'inconvénient de cette méthode réside dans le fait que, plus l'émulsion est fluide, plus il sera difficile d'obtenir une dispersion homogène et stable des nanoparticules. Le pigment mal dispersé dans le produit final se présente alors sous forme d'agrégats qui, une fois appliqués sur la peau, lui confèrent un aspect blanchâtre cosmétiquement indésirable et peu apprécié des utilisateurs. Cet effet est d'autant plus marqué que la concentration en nanopigments dans l'émulsion est élevée. La mauvaise répartition des nanopigments à la surface de la peau est de plus responsable de la mauvaise efficacité du produit.

Il subsiste ainsi un besoin d'un procédé de préparation d'émulsions photoprotectrices contenant des nanopigments permettant, par simple mélange des différents composants, c'est-à-dire de la phase liquide émulsionnée et des nanopigments, l'obtention de compositions relativement fluides faciles et agréables à appliquer, apportant une protection solaire efficace et qui présentent une bonne stabilité dans le temps.

La demanderesse a constaté avec surprise que l'utilisation combinée d'au moins un agent tensioactif choisi dans une famille particulière d'agents tensioactifs décrite plus en détail ci-dessous, et d'au moins un polymère associatif permettait de préparer des compositions solaires fluides émulsifiées contenant des nanopigments sans avoir recours à des techniques d'émulsification haute pression.

On entend par compositions « fluides » selon l'invention des compositions ayant une viscosité, mesurée à 25 °C à l'aide d'un viscosimètre Brookfield avec une aiguille 7, inférieure à 200 mPa.s, de préférence comprise entre 10 et 180 mPa.s

Les agents tensioactifs participant à la résolution du problème de la présente invention sont des agents tensioactifs dimères, appelés le plus souvent agents tensioactifs géminés (en anglais *gemini surfactants*), comportant deux motifs tensioactifs constitués chacun d'une tête hydrophile et d'une queue hydrophobe et reliés l'un à l'autre, au niveau des têtes hydrophiles, par un groupe espaceur.

Les polymères associatifs sont des polymères hydrosolubles capables, dans un milieu aqueux, de s'associer réversiblement entre eux ou avec d'autres molécules.

Leur structure chimique comprend des zones hydrophiles qui leur confèrent leur solubilité dans l'eau, et des zones hydrophobes comportant une chaîne grasse leur permettant de s'associer par interaction hydrophobe entre eux ou avec d'autres molécules comportant des zones hydrophobes.

La présente invention a par conséquent pour objet une composition photoprotectrice contenant,
- en tant que phase liquide, une émulsion huile-dans-eau, émulsionnée par au moins un agent tensioactif dimère comportant deux motifs tensioactifs, identiques ou différents, constitués chacun d'une tête hydrophile et d'une queue hydrophobe et reliés l'un à l'autre, au niveau des têtes hydrophiles, par un groupe espaceur,
- un système photoprotecteur capable de filtrer les rayons UV contenant au moins un nanopigment minéral à base d'oxyde métallique, et
- au moins un polymère associatif non ionique comportant au moins une chaîne grasse en C₈₋₄₀.

Selon un mode de réalisation préféré des compositions photoprotectrices de la présente invention, le système photoprotecteur capable de filtrer les rayons UV contient en outre au moins un filtre UV-A et/ou UV-B organique.

Les agents tensioactifs dimères ou agents tensioactifs géminés utilisés dans la présente invention sont connus. Pour une description détaillée des différentes structures chimiques et de leurs propriétés physico-chimiques, on pourra se référer aux publications suivantes :
Milton J. Rosen, Gemini Surfactants, Properties of surfactant molecules with two hydrophilic groups and two hydrophobic groups, Cosmetics & Toiletries magazine, vol. 113, decembre 1998, pages 49 - 55,
Milton J. Rosen, Recent Developments in Gemini Surfactants, Allured's Cosmetics & Toiletries magazine, juillet 2001, vol 116, n° 7, pages 67 - 70.

On peut citer à titre d'exemples préférés de tensioactifs géminés utilisables dans la présente invention ceux rassemblés dans la demande de brevet allemand DE 199 43 681 A1, à savoir les composés
- de formule (I), décrits dans WO96/14926 : 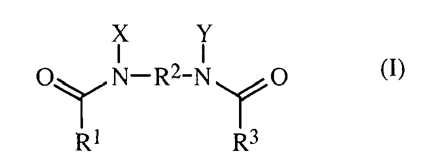 où
   R¹ et R³ représentent un groupe alkyle en C₅₋₂₅, linéaire ou ramifié, saturé ou contenant jusqu'à deux insaturations non voisines,
   R² représente un groupe alkylène en C₁₋₁₂,
   X et Y représentent chacun un groupe (C₂H₄O)ₓ(C₃H₆O)_{y}-RF avec x = 0 - 15, y = 0 - 10, x + y ≥ 1, et RF = -SO₃M, -CH₂-CO₂M, -P(O)(OM)₂, H, -C₃H₆SO₃M, ou un groupe -CH₂(CHOH)₄CH₂OH lorsque x + y = 0, et
   M représente un ion alcalin, (alkyl)ammonium, alcanolammonium, H ou un 1/2 ion alcalinoterreux,
- de formule (II), décrits dans WO96/25388 : 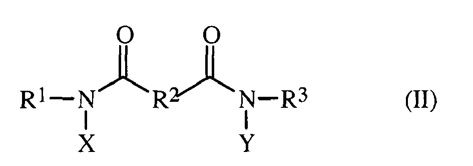 où les symboles ont la même signification que pour la formule (I),
- de formule (III), décrits dans WO97/31890 : 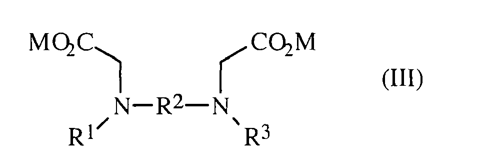 où les symboles ont la signification indiquée pour la formule (I).
- de formule (IV), décrits dans DE 196 22 612 et JP-A 10-175934 : 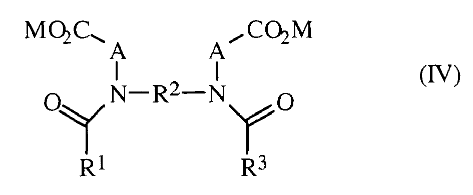 où
   R¹ et R³ représentent un groupe alkyle en C₅₋₂₅, linéaire ou ramifié, saturé ou contenant jusqu'à deux insaturations non voisines,
   R² représente un groupe alkylène en C₁₋₁₂,
   A représente un groupe -CHR⁴-, -CH₂-, -C₂H₄-, -C₃H₆-, -C₄H₈-,
   R⁴ représente le résidu d'un acide aminocarboxylique et
   M représente un ion alcalin, (alkyl)ammonium, alcanolammonium, H ou un 1/2 ion alcalinoterreux,
- de formule (V), décrits dans EP 0 708 079 : 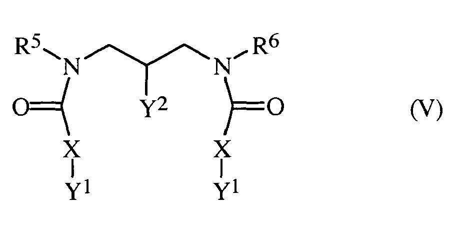 où
   R⁵ et R⁶ représentent un groupe alkyle en C₆₋₃₆, linéaire ou ramifié, saturé ou contenant jusqu'à deux insaturations non voisines,
   X représente un groupe alkylène ou alcénylène comportant de 1 à 6 atomes de carbone, pouvant porter un groupe hydroxyle, acide sulfonique ou acide carboxylique,
   chaque Y¹ représente indépendamment un groupe sulfonate, sulfate, carboxyle ou hydroxyle, un groupe acide sulfurique ou -O-(CO)ₓ-COOH,
- de formule (VI), décrits dans JP-A-8-311003 : 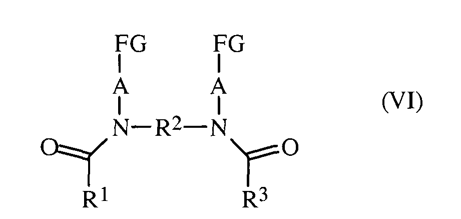 où les symboles ont la signification indiqué pour la formule (IV) et FG représente un groupe -COOM ou -SO₃M,
- de formule (VII), décrits dans JP-A-11-60437 : 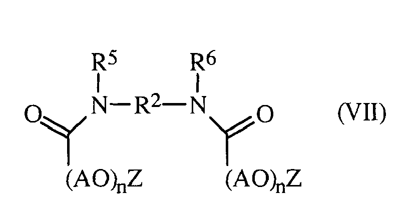 où les substituants ont la signification indiquée pour les formules (IV) et (V),
   AO représente un motif alkylèneoxy, par exemple éthylèneoxy, propylèneoxy et butylèneoxy, n = 1 à 20, les motifs alkylèneoxy pouvant être enchaînés de manière statistique ou par blocs, et
   Z représente un groupe -SO₃M, -C₂H₄SO₃M, -C₃H₆SO₃M, -P(O)(OM)₂, -CH₂-COOM ou -C₂H₄-COOM,
- de formule (VIII), décrits dans EP 0 697 244 : 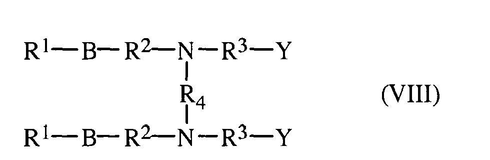 où
   chaque R¹ représente un groupe alkyle en C₅₋₂₅, linéaire ou ramifié, saturé ou contenant jusqu'à deux insaturations non voisines, éventuellement hydroxylé ou perfluoré,
   R² représente un groupe alkylène en C₁₋₁₂, éventuellement hydroxylé,
   B représente un groupe amide, carboxyle ou polyéther,
   R³ représente un groupe alkyle en C₁₋₁₂ éventuellement hydroxylé, un groupe R⁷-D-R⁷ ou un groupe polyéther, où R⁷ représente un groupe alkylène en C₁₋₆ éventuellement hydroxylé,
   D représente un groupe -O-, -S- ou -NR⁸-,
   R⁴ représente un groupe alkylène ou alkylarylène comportant de 1 à 12 atomes de carbone, éventuellement hydroxylé, ou un groupe R⁹-D¹-R⁹,
   R⁸ représente un groupe alkyle en C₁₋₁₂ éventuellement hydroxylé, un atome d'hydrogène ou un groupe R⁹-D¹-R⁹,
   R⁹ représente un groupe alkylène en C₁₋₆ éventuellement hydroxylé, ou un groupe aryle,
   D¹ = -O-, -S-, -SO₂-, -C(O)-, -O(R⁷-O)ₓ-, (R¹⁰)ₜ[N(R¹⁰)]_{z} ou un groupe aryle,
   R¹⁰ représente un groupe alkyle en C₁₋₁₂ éventuellement substitué ou un atome d'hydrogène ou un groupe aryle,
   t et z valent indépendamment chacun un nombre compris entre 1 et 4, et
   chaque Y représente indépendamment un groupe -SO₃H, -OSO₃H, -OP(O)(OH)₂, -P(O)(OH)₂, -COOH, -CO₂-C₆H₄-SO₃H et les sels correspondants,
- de formule (IX), décrits dans EP 0 697 245 : 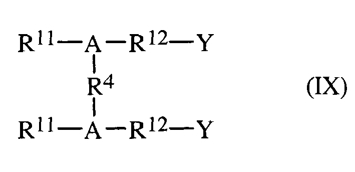 où chaque R¹¹ représente un groupe alkyle en C₅₋₁₂, linéaire ou ramifié, saturé ou comportant jusqu'à deux insaturations non voisines, éventuellement hydroxylé ou perfluoré, ou un groupe R¹⁴-B-R²,
   R¹⁴ représente un groupe alkyle en C₁₋₁₂, linéaire ou ramifié, saturé ou comportant jusqu'à deux insaturations non voisines, éventuellement hydroxylé,
   R¹² représente un groupe alkyle en C₁₋₁₂, linéaire ou ramifié, saturé ou comportant jusqu'à deux insaturations non voisines, éventuellement hydroxylé ou un groupe amide, carboxyle, polyéther ou R⁹-D¹-R⁹, et
   A représente un groupe -CR²= ou -N=, sous réserve que, lorsque A représente un groupe -N=, alors R¹¹ est un groupe R¹⁴-B-R².
   R², R⁴, B, R⁹ et D¹ ayant la signification indiquée pour la formule (VIII).
- de formule (X), décrits dans DE 42 27 391 et DE 196 08 117 : 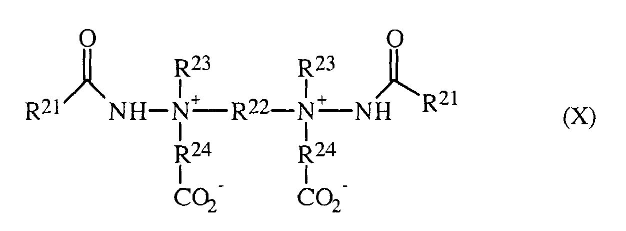 où
   chaque R²¹ représente un groupe alkyle en C₅₋₂₃, linéaire ou ramifié, saturé ou comportant jusqu'à 2 insaturations non-voisines,
   R²² et chaque R²⁴ représentent un groupe alkylène en C₁₋₆, et
   Chaque R²³ représente un groupe méthyle, éthyle, propyle ou polyéther.
- de formule (XI), décrits dans US 5 863 886 : 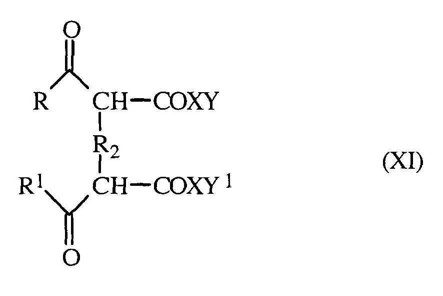 où
   R et R¹ représente chacun un groupe alkyle en C₅₋₃₀, linéaire ou ramifié, saturé ou comportant jusqu'à 2 insaturations non-voisines, éventuellement hydroxylé ou perfluoré,
   R² représentent un groupe alkylène en C₁₋₁₀ ou arylène éventuellement hydroxylés, un groupe polyéther, -S-, -SO₂-, -O-, -S-S-, -O-R⁵-O- ou -S-R⁵-S-, ou une liaison directe entre les deux atomes de carbone en α,
   R⁵ représente un groupe alkylène, arylène ou alkylarylène en C₁₋₁₀, -N(R⁶)- ou -(NR⁶)-R⁷-(NR⁶)-,
   R⁶ représente un groupe alkyle en C₁₋₆,
   R⁷ représente un groupe alkylène en C₁₋₆,
   ou R⁶ et R⁷ forment un hétérocycle,
   X représente un groupe polyéther, -O- ou -NZ- avec Z = H, ou alkyle, aryle ou alkylaryle en C₁₋₁₀,
   Y et Y¹ représentent chacun indépendamment un atome d'hydrogène ou un groupe -CH₂-COOH éventuellement salifié, un résidu carbohydrate comportant au moins deux groupes hydroxyle, tels que érythrose, thréose, ribose, arabinose, xylose, fructose, lyxose, allose, altrose, glucose, mannose, galactose, et des mélanges,
- de formule (XII) : 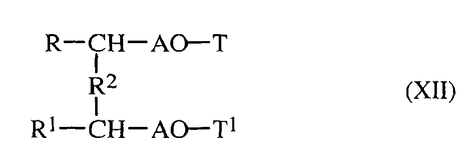 où les symboles ont la signification indiquée pour la formule (XI) et AO représente un groupe -C(O)-, -C(O)-[-O(R⁴O)ₓ], -CH₂-[O(R⁴O)ₓ)], ou -CH₂-O,
   R⁴ représente un groupe alkylène en C₂₋₄,
   T et T¹ représentent indépendamment chacun un groupe -OM, -H, -CH₃, -C₂H₅, -SO₃M, -CH₂COOM, -C₂H₄-COOM, -C₃H₆-CO₃M, -O-, P(O)(OM)₂ et M représente un ion alcalin ou un demi-ion alcalinoterreux, ou un ion mono-, di- ou trialcanolammonium ou un proton.
- de formule (XIII), décrits dans WO 96/16930 : 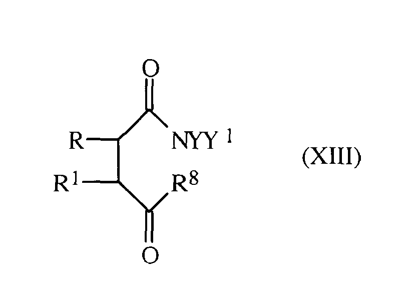 où les symboles ont la signification indiquée pour les formules (XI) et (XII) et R⁸ représente un groupe NYY¹, -O(R⁴O)ₓH ou -O(R⁴O)ₓ-C(O)-CHR-CHR¹-C(O)NYY¹.
- de formule (XIV), décrits dans WO 96/25384 : 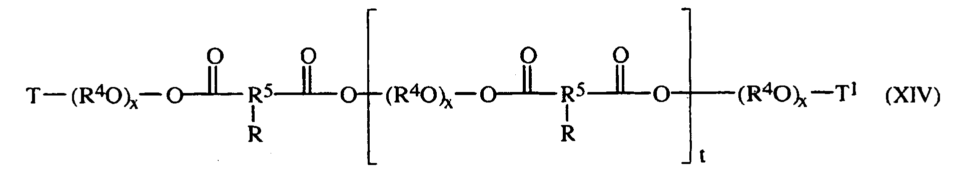
où les symboles ont la signification indiquée pour les formules (XI) à (XIII) et t représente un nombre entier allant de 1 à 100, de préférence de 1 à 4.

Parmi les agents tensioactifs dimères ci-dessus, on préfère en particulier les tensioactifs anioniques, et en particulier ceux correspondant à la formule (I) ci-dessus. Dans cette famille d'agent tensioactifs, on préfère en particulier ceux dans lesquels R¹ et R³ sont identiques et représentent chacun un groupe alkyle linéaire en C₈₋₁₆, R² représente un groupe alkylène en C₂₋₈, X et Y représentent chacun un groupe -(C₂H₄O)ₓ-RF avec x = 10 - 15 et RF = -SO₃M, où M est un atome de métal alcalin.

Un agent tensioactif géminé préféré de cette famille est un composé anionique de formule : 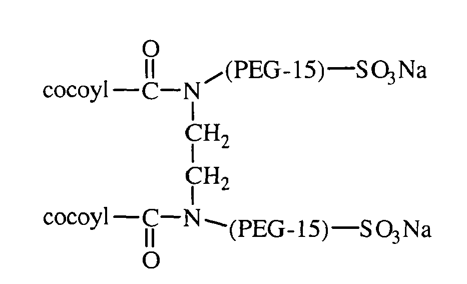 nom INCI : sodium dicocoylethylenediamine PEG-15 sulfate.

On peut utiliser par exemple cet agent tensioactif géminé commercialisé par la société SASOL sous la dénomination CERALUTION®, et notamment les produits suivants :
- Ceralution® H : Behenyl Alcohol, Glyceryl Stearate, Glyceryl Stearate Citrate et Sodium Dicocoylethylenediamine PEG-15 Sulfate,
- Ceralution® F : Sodium Lauroyl Lactylate et Sodium Dicocoylethylènediamine PEG-15 Sulfate.
- Ceralution® C : Aqua, Capric/Caprylic triglyceride, Glycerin, Ceteareth-25, Sodium Dicocoylethylenediamine PEG-15 Sulfate, Sodium Lauroyl Lactylate, Behenyl Alcohol, Glyceryl Stearate, Glyceryl Stearate Citrate, Gum Arabic, Xanthan Gum, Phenoxyethanol, Methylparaben, Ethylparaben, Butylparaben, Isobutylparaben (dénominations INCI)

La concentration du ou des agents tensioactifs géminés utilisés dans la présente invention est de préférence comprise entre 0,001 et 8 %, de préférence entre 0,01 et 4 % et en particulier entre 0,05 et 3 %, rapporté au poids total de la composition photoprotectrice.

Les polymères associatifs utilisés dans la présente invention sont de type non ionique.

Leur concentration en poids dans la composition photoprotectrice selon l'invention peut varier d'environ 0,01 à 10 % du poids total de la composition. Plus préférentiellement, cette concentration est comprise entre 0,1 à 5 % du poids total de la composition.

Les polymères associatifs non-ioniques utilisés dans la présente invention sont choisis de préférence parmi :
(1) les celluloses, en particulier les hydroxyéthylcelluloses, modifiées par des groupements comportant au moins une chaîne grasse. On peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyle en C₁₆) vendu par la société AQUALON, ou le produit BERMOCOLL EHM 100® vendu par la société BEROL NOBEL,
   - les hydroxyéthylcelluloses modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
(2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
(3) les copolymères de vinylpyrrolidone et de monomères hydrophobes à chaîne grasse dont on peut citer à titre d'exemple :
   - les produits ANTARON V216® ou GANEX V216® (copolymère vinylpyrrolidone/hexadécène) vendu par la société I.S.P.
   - les produits ANTARON V220® ou GANEX V220® (copolymère vinylpyrrolidone/eicosène) vendu par la société I.S.P.
(4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
(5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
(6) les polyéther-polyuréthannes comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
   De préférence, les polyéther-polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
   Les polyéther-polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
   Les polyéther-polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
   Par extension figurent aussi parmi les polyéther-polyuréthanes non-ioniques à chaîne grasse, ceux dont les séquences hydrophiles sont liées aux séquences lipophiles par d'autres liaisons chimiques.
   A titre d'exemples de polyéther-polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.
   On peut également citer le produit ELFACOS T210® à chaîne alkyle en C₁₂₋₁₄ et le produit ELFACOS T212® à chaîne alkyle en C₁₈ de chez AKZO.
   Le produit DW 1206B® de chez ROHM & HAAS à chaîne alkyle en C₂₀ et à liaison uréthanne, proposé à 20 % en matière sèche dans l'eau, peut aussi être utilisé.
   On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.
   Les polyéther-polyuréthanes utilisables selon l'invention sont en particulier ceux décrits dans l'article de G. Fonnum, J. Bakke et Fk. Hansen - Colloid Polym. Sci 271, 380.389 (1993).
   Plus particulièrement encore, selon l'invention, on préfère utiliser un polyéther-polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.
   De tels polyéther-polyuréthanes sont vendus notamment par la société ROHM & HAAS sous les appellations Aculyn 46® et Aculyn 44®. L'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%). L'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%).
(7) les copolymères de PEG-180, de tétramethoxyméthylglycouril et de laureth-50 ou d'octoxynol-40 (noms INCI), proposés sous les dénominations PURE THIX® 1450 et PURE THIX® 1451 par la société SUD-CHEMIE.

Le système photoprotecteur utilisé dans les compositions photoprotectrices de la présente invention comprend :
- au moins un nanopigment minéral à base d'oxyde métallique et,
- de préférence, en outre au moins un filtre UV-A et/ou UV-B organique.

Les filtres UV-A et/ou UV-B organiques susceptibles d'être utilisés peuvent être choisis notamment parmi les dérivés cinnamiques, les dérivés de dibenzoylméthane, les dérivés salicyliques, les dérivés du benzylidènecamphre, les dérivés de triazine tels que ceux décrits dans les brevets ou demandes de brevet US 4 367 390, EP 0 863 145, EP 0 517 104, EP 0 570 838, EP 0 796 851, EP 0 775 698, EP 0 878 469, EP 0 933 376, EP 0 507 691, EP 0 507 692, EP 0 790 243, EP 0 944 624 et US 4 724 137 ; les dérivés de la benzophénone ; les dérivés de β,β'-diphénylacrylate ; les dérivés de benzotriazole, les dérivés de benzalmalonate, les 4,4-diarylbutadiènes, les dérivés de bis-benzoxazolyle tels que décrits dans les brevets EP 669 323 et US 2 463 264, les dérivés de méthylène bis-(hydroxyphénylbenzotriazole) tels que décrits dans les brevets US 5 237 071, US 5 166 355, GB 2 303 549, DE 197 26 184 et EP 893 119, les dérivés de phénylbenzimidazole ; les dérivés anthraniliques ; les dérivés d'imidazolines ; les dérivés de l'acide p-aminobenzoïque, les polymères hydrocarbonés filtres et les silicones filtres tels que ceux décrits notamment dans la demande WO 93/04665 ; les dimères dérivés d'α-alkylstyrène tels que décrits dans la demande DE 198 55 649 ; ainsi que les mélanges de ces filtres.

On peut citer à titre d'exemples de tels filtres actifs dans l'UV-A et/ou UV-B, les composés suivants désignés par leur nom INCI, ainsi que leurs mélanges :
dérivés d'acide para-aminobenzoïque :
   - PABA
   - Ethyl PABA
   - Ethyl Dihydroxypropyl PABA
   - Ethylhexyl Dimethyl PABA vendu notamment sous le nom commercial ESCALOL 507 par ISP,
   - Glyceryl PABA,
   - PEG-25 PABA vendu sous le nom commercial UVINUL P25 par BASF,
dérivés salicyliques :
   - Homosalate vendu sous le nom commercial EUSOLEX HMS par RONA/EM INDUSTRIES,
   - Ethylhexyl Salicylate vendu sous le nom commercial NEO HELIOPAN OS par HAARMANN ET REIMER,
   - Dipropyleneglycol Salicylate vendu sous le nom commercial DIPSAL par SCHER,
   - TEA Salicylate vendu sous le nom commercial NEO HELIOPAN TS par HAARMANN ET REIMER,
dérivés de dibenzoylméthane :
   - Butyl Methoxydibenzoylmethane vendu notamment sous le nom commercial PARSOL 1789 par HOFFMANN LAROCHE,
   - Isopropyl Dibenzoylmethane,
dérivés cinnamiques :
   - Ethylhexyl Methoxycinnamate vendu notamment sous le nom commercial PARSOL MCX par HOFFMANN LAROCHE,
   - Isopropyl Methoxycinnamate
   - Isoamyl Methoxycinnamate vendu sous le nom commercial NEO HELIOPAN E 1000 par HAARMANN ET REIMER,
   - Cinoxate,
   - DEA Methoxycinnamate,
   - Diisopropyl Methylcinnamate,
   - Glyceryl Ethylhexanoate Dimethoxycinnamate
dérivés de β,β-diphénylacrylate :
   - Octocrylene vendu notamment sous le nom commercial UVINUL-539 par BASF
   - Etocrylene vendu notamment sous le nom commercial UVINUL N35 par BASF
dérivés de benzophénone :
   - Benzophenone-1 vendue sous le nom commercial UVINUL 400 par BASF,
   - Benzophenone-2 vendue sous le nom commercial UVINUL D-50 par BASF,
   - Benzophenone-3 ou Oxybenzone vendue sous le nom commercial UVINUL M-40 par BASF,
   - Benzophenone-4 vendue sous le nom commercial UVINUL MS-40 par BASF,
   - Benzophenone-5,
   - Benzophenone-6 vendue sous le nom commercial HELISORB 11 par NORQUAY,
   - Benzophenone-8 vendue sous le nom commercial SPECTRA-SORB UV-24 par AMERICAN CYANAMID
   - Benzophenone-9 vendue sous le nom commercial UVINUL DS-49 par BASF,
   - Benzophénone-12
   - 2-(4-diéthylamino-2-hydroxybenzoyl)-benzoate de n-hexyle
dérivés du benzylidène camphre :
   - 3-benzylidene camphor fabriqué sous le nom MEXORYL SD par CHIMEX
   - 4-methylbenzylidene camphor vendu sous le nom EUSOLEX 6300 par MERCK
   - Benzylidene Camphor Sulfonic Acid fabriqué sous le nom MEXORYL SL par CHIMEX,
   - Camphor Benzalkonium Methosulfate fabriqué sous le nom MEXORYL SO par CHIMEX,
   - Terephthalylidene Dicamphor Sulfonic Acid fabriqué sous le nom MEXORYL SX par CHIMEX,
   Polyacrylamidomethyl Benzylidene Camphor fabriqué sous le nom MEXORYL SW par CHIMEX,
dérivés de phénylbenzimidazole :
   - Phenylbenzimidazole Sulfonic Acid vendu notamment sous le nom commercial EUSOLEX 232 par MERCK
   - Benzimidazilate vendu sous le nom commercial NEO HELIOPAN AP par HAARMANN ET REIMER,
   - Disodium Phenyl Dibenzimidazole Tetra-sulfonate vendu sous le nom commercial NEO HELIOPAN AP par Haarmann et Reimer
dérivés de triazine :
   - Anisotriazine vendue sous le nom commercial TINOSORB S par CIBA GEIGY
   - Ethylhexyl triazone vendue notamment sous le nom commercial UVINUL T150 par BASF,
   - Diéthylhexyl Butamido Triazone vendue sous le nom commercial UVASORB HEB par SIGMA 3V,
   - 2,4,6-tris-(4'-amiobenzalmalonate de diisobutyle)-s-triazine
dérivés de benzotriazole :
   - Drometrizole Trisiloxane vendu sous le nom commercial SILATRIZOLE par RHODIA CHIMIE,
   - Methylene bis-Benzotriazolyl Tetramethylbutylphenol vendu sous forme solide sous le nom commercial MIXXIM BB/1 00 par FAIRMOUNT CHEMICAL ou sous forme micronisée en dispersion aqueuse sous le nom commercial TINOSORB M par Ciba Specialty Chemicals.
dérivés anthraniliques :
   Menthyl Anthranilate vendu sous le nom commercial NEO HELIOPAN MA par HAARMAN ET REIMER,
dérivés d'imidazolines :
   Ethylhexyl Dimethoxybenzylidene Dioxoimidazoline Propionate
dérivés du benzalmalonate :
   Polyorganosiloxane à fonctions benzalmalonate tel que le polysilicone-15 vendu sous le nom commercial PARSOL SLX par HOFFMANN LAROCHE
4,4-diarylbutadiènes :
   1,1-dicarboxy-(2,2'-diméthylpropyl)-4,4-diphénylbutadiène

Les filtres UV organiques plus particulièrement préférés sont choisis parmi les composés suivants (noms INCI) :
Ethylhexyl Salicylate,
Ethylhexyl Methoxycinnamate,
Octocrylene,
Butyl Methoxydienzoylmethane
Phenylbenzimidazole Sulfonic Acid,
Terephthalylidene Dicamphor Sulfonic Acid,
Benzophenone-3,
Benzophenone-4,
Benzophenone-5,
2-(4-diéthylamino-2-hydroxybenzoyl)-benzoate de n-hexyle
4-Methylbenzylidene camphor
Terephtalylidene Dicamphor Sulfonic Acid
Disodium Phenyl Dibenzimidazole Tetrasulfonate
2,4,6-tris-(4'-aminobenzalmalonate de diisobutyl)-s-triazine
Anisotriazine,
Ethylhexyl Triazone,
Diethylhexyl Butamido Triazone,
Methylene bis-benzotriazolyl tetramethylbutylphénol
Drometrizole Trisiloxane,
Polysilicone-15,
1,1-dicarboxy-(2,2'diméthylpropyl)-4,4-diphénylbutadiène
et leurs mélanges.

Les nanopigments minéraux à base d'oxydes métalliques utilisés dans la présente invention sont des poudres constituées de particules ayant une taille élémentaire moyenne généralement comprise entre 5 nm et 100 nm, de préférence entre 10 et 50 nm. Les oxydes métalliques formant ces nanopigments peuvent être choisis notamment parmi les oxydes de titane, de fer, de zinc, de zirconium et de cérium. On préfère tout particulièrement parmi ces oxydes l'oxyde de titane, amorphe ou sous forme cristallisée (rutile et/ou anatase). Ces nanopigments peuvent être revêtus d'un enrobage, constitué par exemple d'alumine et/ou de stéarate d'aluminium, ou d'un silane ou polymère siliconé.

Ces nanopigments et leur utilisation en tant qu'agents photoprotecteurs sont connus et décrits par exemple dans les demandes EP 518772 et EP 518773.

La concentration des nanopigments et des filtres UV organiques dépend bien entendu de l'indice de protection souhaité. Elle est généralement comprise entre 0,5 et 10 % en poids pour les nanopigments, et entre 0,1 et 30 %, de préférence entre 0,5 et 15 % pour les filtres UV organiques, toutes ces concentrations étant rapportées au poids total de la composition photoprotectrice.

La nature de la phase grasse servant à la préparation des émulsions photoprotectrices selon l'invention n'est pas déterminante et elle peut ainsi être constituée par tous les composés connus susceptibles de servir pour la fabrication d'émulsions huile-dans-eau. En particulier, ces composés peuvent être choisis parmi les différents corps gras, les huiles d'origine végétale, animale ou minérale, les cires naturelles ou synthétiques et analogues.

Parmi les huiles pouvant rentrer dans la composition de la phase grasse, on peut notamment citer :
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-α-oléfines.

Comme autres huiles utilisables dans les émulsions selon l'invention, on peut encore citer les benzoates d'alcools gras en C₁₂-C₁₅ (Finsolv TN de FINETEX), les alcools gras tels que l'alcool laurylique, cétylique, myristylique, stéarylique, palmitique, oléylique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en C₁₀-C₁₈, les huiles fluorées et perfluorées, la lanoline, la lanoline hydrogénée, la lanoline acétylée et enfin les huiles de silicones, volatiles ou non.

La phase huileuse de l'émulsion représente de préférence de 0,1 à 45 % et en particulier de 5 à 30 % du poids total de la phase liquide.

Bien entendu, la phase grasse peut également contenir un ou plusieurs adjuvants cosmétiques lipophiles classiques, notamment ceux qui sont déjà utilisés de manière habituelle dans la fabrication et l'obtention des compositions cosmétiques anti-solaires.

De manière classique, la phase aqueuse continue peut être constituée par de l'eau, ou un mélange d'eau et d'alcool(s) polyhydrique(s) comme par exemple le glycérol, le propylèneglycol et le sorbitol, ou bien encore un mélange d'eau et d'alcool(s) inférieur(s) hydrosoluble(s) tels que l'éthanol, l'isopropanol ou le butanol et elle peut bien entendu en outre contenir des adjuvants cosmétiques classiques hydrosolubles.

Les compositions photoprotectrices selon l'invention peuvent également contenir des agents de bronzage et/ou de brunissage artificiels de la peau (agents autobronzants).

Les agents autobronzants sont généralement choisis parmi les composés mono- ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA. La DHA peut être utilisée sous forme libre et/ou encapsulée par exemple dans des vésicules lipidiques telle que des liposomes, notamment décrits dans la demande WO 97/25970.

Les agents autobronzants mono- ou polycarbonylés, lorsqu'ils sont présents, sont généralement utilisés à raison de 0,1 à 10 % en poids par rapport au poids total de la composition, et de préférence de 0,2 à 8 % en poids par rapport au poids total de la composition.

Les compositions de la présente invention peuvent comprendre en outre des adjuvants cosmétiques classiques notamment choisis parmi les épaississants et/ou agents gélifiants, les agents adoucissants, les humectants, les opacifiants, les stabilisants, les émollients, les silicones, les agents répulsifs contre les insectes, les parfums, les conservateurs, les charges, les pigments, les propulseurs, les agents alcalinisants ou acidifiants ou tout autre ingrédient habituellement utilisé dans le domaine cosmétique et/ou dermatologique.

Bien entendu, l'homme de l'art veillera à choisir le ou les éventuels composés complémentaires cités ci-dessus et/ou leurs quantités de manière telles que les propriétés avantageuses attachées intrinsèquement aux émulsions conformes à l'invention ne soient pas, ou substantiellement pas, altérées par la ou les adjonctions envisagées.

L'invention a également pour objet un procédé de préparation de telles compositions photoprotectrices émulsifiées, comprenant l'homogénéisation, à une température comprise entre 40 et 70 °C et à l'aide d'un mixer ou d'un homogénéisateur de type rotor-stator, d'une phase grasse contenant éventuellement un agent tensioactif géminé, éventuellement un ou plusieurs filtres UV organiques, et éventuellement un ou plusieurs nanopigment(s), et d'une phase aqueuse contenant éventuellement un agent tensioactif géminé, éventuellement un ou plusieurs filtres UV organiques et éventuellement un ou plusieurs nanopigment(s), étant entendu qu'au moins une de ces phases contient un agent tensioactif géminé, un polymère associatif étant incorporé soit dans la phase aqueuse ou la phase grasse avant homogénéisation, soit dans l'émulsion fine obtenue, après refroidissement jusqu'à une température comprise entre 30 et 40 °C.

Le ou les nanopigments peuvent également être incorporé(s) sous forme pulvérulente dans l'émulsion huile-dans-eau contenant le polymère associatif. Dans ce cas, il est nécessaire de soumettre la composition à une deuxième étape d'homogénéisation.

Le procédé de la présente invention se distingue par le fait qu'il peut être mis en oeuvre sans aucune étape d'homogénéisation haute pression.

### Exemple 1

| | Concentration (% en poids) |
|---|---|
| Phase A | |
| Céralution® H | 2,5 |
| C12-15 alkyl benzoate | 11,15 |
| Isohexadécane | 5,6 |
| Octocrylene | 9 |
| Butyl methoxydibenzoylmethane | 2,5 |
| Drometrizole trisiloxane | 0,75 |
| Conservateur | q.s. |

| Phase B | |
|---|---|
| Eau | q.s.p. 100 |
| Disodium ethylendiamine tetramethylene phosphonate | 0,1 |
| Terephtalylidene dicamphor sulfonic acid | 0,75 |
| Cétylhydroxyéthylcellulose | 0,5 |
| (NATROSOL PLUS GRADE 330CS vendu par Aqualon) | 5 |
| Triethanolamine | q.s. |

| Phase C | |
|---|---|
| Dioxyde de titane | 5 |

| Phase D | |
|---|---|
| Conservateur | |

Mode opératoire pour la préparation d'une composition photoprotectrice selon l'invention : On chauffe séparément les phases A et B à une température d'environ 70 °C, puis on soumet ces deux phases réunies à une homogénéisation à l'aide d'un appareil à rotor-stator. On ajoute le dioxyde de titane (phase C) sous forme pulvérulente dans l'émulsion obtenue et l'on procède à une deuxième homogénéisation. Après refroidissement à 25 °C, on ajoute l'agent conservateur (phase D).

### Exemple 2

| | Concentration (% en poids) |
|---|---|
| Phase A | |
| Céralution® H | 1 |
| Caprylic/capric triglyceride | 5 |
| Cyclopentasiloxane | 4 |
| C12-15 alkyl benzoate | 2,5 |
| Triethyl citrate | 4 |
| Isotrideceth-12 | 2 |
| Octocrylene | 9 |
| Butyl methoxydibenzoylmethane | 2,5 |

| Phase B | |
|---|---|
| Eau | 6 |
| Ceralution® F | 1,5 |
| Gomme de xanthane | 0,1 |
| Glycérine | 6 |

| Phase C | |
|---|---|
| Eau | q.s.p. 100 |
| Copolymer vinylpyrrolidone/eicosene | |
| (ANTARON V220® vendu par la société I.S.P.) | 1 |
| Alcool dénaturé | 6 |
| Terephtalylidene dicamphor sulfonic acid | 0,75 |
| Triéthanolamine | q.s. |

| Phase D | |
|---|---|
| Dioxyde de titane enrobé de triméthoxycaprylylsilane | 5 |

Mode opératoire pour la préparation d'une composition photoprotectrice selon l'invention : On chauffe séparément les phases A et B à une température d'environ 60 °C, on ajoute le dioxyde de titane (phase D) à la phase A en agitant avec un appareil à rotor-stator, on verse le mélange phase A+D dans la phase B, puis on procède à l'homogénéisation de l'ensemble. On laisse le mélange obtenu refroidir jusqu'à une température d'environ 40 °C et l'on y incorpore la phase C par une deuxième étape d'homogénéisation.

### Exemple 3

| | Concentration (% en poids) |
|---|---|
| Phase A | |
| Céralution® H | 1 |
| Caprylic/capric triglyceride | 5 |
| C12-15 alkyl benzoate | 6,5 |
| Dicaprylyl carbonate | 4 |
| Isotrideceth-12 | 2 |
| Octocrylene | 9 |
| Butyl methoxydibenzoylmethane | 2,5 |

| Phase B | |
|---|---|
| Eau | 6 |
| Ceralution® F | 1,5 |
| Glycérine | 6 |

| Phase C | |
|---|---|
| Eau | q.s.p. 100 |
| PEG-150/steraryl alcohol/SMDI Copolymer (15 %)/Aqua | |
| (ACULYN 46® vendu par ROHM et HAAS) | 3 |
| Terephtalylidene dicamphor sulfonic acid | 0,75 |
| Triéthanolamine | q.s. |

| Phase D | |
|---|---|
| Dioxyde de titane enrobé avec | |
| hydroxyde d'aluminium et acide stéarique | 5 |

| Phase E | |
|---|---|
| Phénoxyéthanol et methylparaben et ethylparaben et butylparaben et isobutylparaben et propylparaben | 1 |

Mode opératoire pour la préparation d'une composition photoprotectrice selon l'invention : On chauffe séparément les phases A et B à une température d'environ 60 °C, on incorpore la phase D dans la phase A chauffée en agitant à l'aide d'un appareil à rotor-stator, puis on verse le mélange phase A+D dans la phase B. Après homogénéisation du mélange, on laisse refroidir jusqu'à 40 °C et l'on ajoute la phase C. Après une nouvelle étape d'homogénéisation, on laisse refroidir à 25 °C et l'on ajoute l'agent conservateur (phase E).

## Revendications

1. Composition photoprotectrice, destinée à la protection de la peau et/ou des cheveux contre le rayonnement UV, contenant,
• en tant que phase liquide, une émulsion huile-dans-eau, émulsionnés par au moins un agent tensioactif dimère comportant deux motifs tensioactifs, identiques on différents, constitués chacun d'une tête hydrophile et d'une queue hydrophobe et reliés l'un à l'autre, au niveau des têtes hydrophiles, par un groupe espaceur,
• un système photoprotecteur capable de filtrer les rayons UV contenant au moins un nanopigment minéral à base d'oxyde métallique, et
• au moins un polymère associatif C₈₋₄₀. non-ionique comportant au moins une chaîne grasse en

2. Composition photoprotectrice selon la revendication 1, **caractérisée par le fait que** le système photoprotecteur capable de filtrer les rayons UV contient en outre an moins un filtre UV-A et/ou UV-B organique.

3. Composition photoprocectrice selon la revendication 1 ou 2, **caractérisée par le fait que** l'agent tensioactif dimère est choisi parmi ceux
• de formule (I) 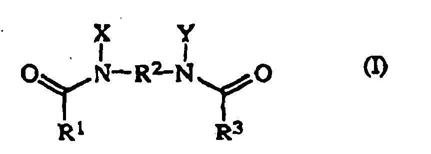 où
R¹ et R³ représentent un groupe alkyle en C₅₋₂₅, linéaire ou ramifié, saturé ou contenant jusqu'à deux insaturations non voisines,
R² représente un groupe alkylène en C₁₋₁₂,
X et Y représentent chacun un groupe (C₂H₄O)ₓ(C₃H₆O)_{y}-RF avec x = 0 - 15, y = 0-10, x + y ≥ 1, et RF = -SO₃M, -CH₂-CO₂M, -P(O)(OM)₂, H, -C₃H₆SO₃M, ou un groupe -CH₂(CHOH)₄CH₂OH lorsque x + y = 0, et
M représente un ion alcalin, (alkyl)ammonium, alcanolammonium, H ou un 1/2 ion alcalmoterreux,
• de formule (II) 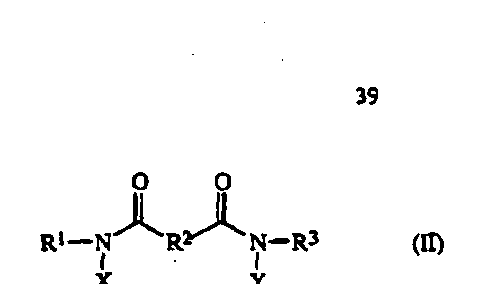 où les symboles ont la même signification que pour la formule (I),
• de formule (III), 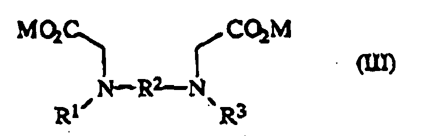 où les symboles ont la signification indiquée pour la formule (I).
• de formule (IV) 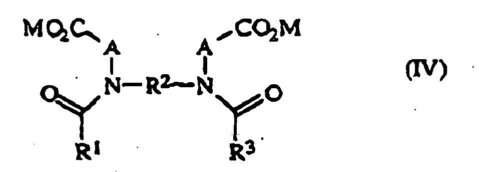 où
R¹ et R³ représentent un groupe alkyle en C₅₋₂₅, linéaire ou ramifié, saturé ou contenant jusqu'à deux insaturations non voisines,
R² représente un groupe alkylène en C₁₋₁₂,
A représente un groupe -CHR⁴-, -CH₂-, -C₂H₄-, -C₃H₄-, -C₄H₈-,
R⁴ représente le résidu d'un acide aminocarboxylique et
M représente un ion alcalin, (alkyl)ammonium, alcanolammonium, H ou un 1/2 ion alcalinoterreux,
• de formule (V) 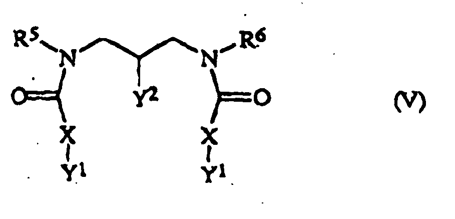 où
R⁵ et R⁶ représentent un groupe alkyle en C₆₋₃₆, linéaire ou ramifié, saturé ou contenant jusqu'à deux insaturations non voisines,
X représente un groupe alkylène ou alcénylène comportant de 1 à 6 atomes de carbone, pouvant porter un groupe hydroxyle, acide sulfonique ou acide carboxylique,
chaque Y¹ représente indépendamment un groupe sulfonate, sulfate, carboxyle ou hydroxyle, un groupe acide sulfurique ou -O-(CO)ₓ-COOH,
• de formule (VI) 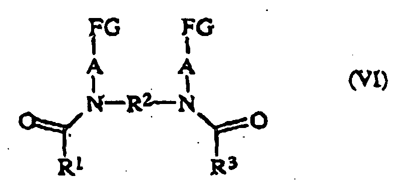 où les symboles ont la signification indiqué pour la formule (IV) et FG représente un groupe -COOM ou -SO₃M,
• de formule (VII) 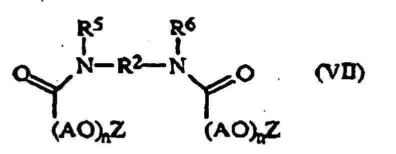 où les substituants ont la signification indiquée pour les formules (IV) et (V),
AO représente un motif alkylèneoxy, par exemple éthylèneoxy, propylèneoxy et butylèneoxy, n = 1 à 20, les motifs alkylèncoxy pouvant être enchaînés de manière statistique ou par blocs, et
Z représente un goupe -SO₃M, -C₂H₄SO₃M, -C₃H₆SO₃M, -P(O)(OM)₂, -CH₂-COOM ou -C₂H₄-COOM,
• de formule (VIII) 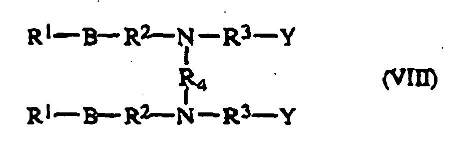 où
chaque R¹ représente un groupe alkyle en C₅₋₂₅, linéaire ou ramifié, saturé ou contenant jusqu'à deux insaturations non voisines, éventuellement hydroxylé ou perfluoré,
R² représente un groupe alkylène en C₁₋₁₂, éventuellement hydroxylé,
B représente un groupe amide, carboxyle ou polyéther,
R³ représente un groupe alkyle en C₁₋₁₂ éventuellement hydroxylé, un groupe R⁷-D-R⁷ ou un groupe polyéther, où R⁷ regrésente un groupe alkylène en C₁₋₆ éventuellement hydroxylé,
D représente un groupe -O-, -S- ou -NR⁸-,
R⁴ représente un groupe alkyléne ou alkylarylène comportant de 1 à 12 atomes de carbone, éventuellement hydroxylé, ou un groupe R⁹-D¹-R⁹,
R⁸ représente un groupe alkyle en C₁₋₁₂ éventuellement hydroxylé, un atome d'hydrogène ou un groupe R⁹-D¹-R9,
R⁹ représente un groupe alkylène en C₁₋₆ éventuellement hydroxyle, ou un groupe aryle,
D¹ = -O-, -S-, -SO₂-, -C(O)-, -O(R⁷-O)ₓ-, (R¹⁰)ₜ[N(R¹⁰)]_{z} ou un groupe aryle,
R¹⁰ représente un groupe alkyle en C₁₋₁₂ éventuellement substitué ou un atome d'hydrogène ou un groupe aryle,
t et z valent indépendamment chacun un nombre compris entre 1 et 4, et
chaque Y représente indépendamment un groupe -SO₃H, -OSO₃H, -OP(O)(OH)₂, -P(O)(OH)₂, -COOH, -CO₂-C₆H₄-SO₃H et les sels correspondants,
• de formule (IX) 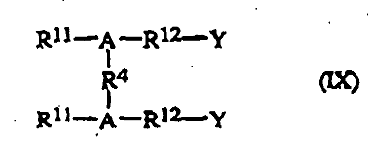
où chaque R¹¹ représente un groupe alkyle en C₅₋₁₂, linéaire ou ramifié, saturé ou comportant jusqu'à deux insaturations non voisines, éventuellement hydroxyle ou perfluoré, ou un groupe R¹⁴-B-R²,
R¹⁴ représente un group alkyle en C₁₋₁₂, linéaire ou ramifié, saturé ou comportant jusqu'à deux insaturations non voisines, éventuellement hydroxylé,
R¹² représente un groupe alkyle en C₁₋₁₂, linéaire ou ramifié, saturé ou comportant jusqu'à deux insaturations non voisines, éventuellement hydroxylé ou un groupe amide, carboxyle, polyéther ou R⁹-D¹-R⁹, et
A représente un groupe -CR²= ou -N=, sous réserve que, lorsque A représente un groupe -N=, alors R¹¹ est un groupe R¹⁴-B-R².
R², R⁴, B, R⁹ et D¹ ayant la signification indiquée pour la formule (VIII).
• de formule (X) 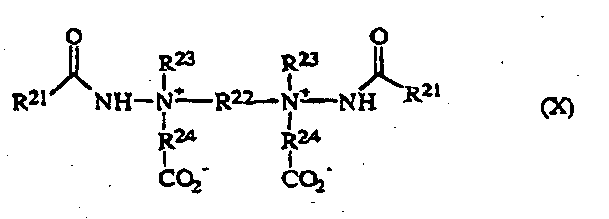 où
chaque R²¹ représente un groupe alkyle en C₅₋₂₃, linéaire ou ramifié, saturé ou comportant jusqu'à 2 insaturations non-voisines,
R²² et chaque R²⁴ représentent un groupe alkylène en C₁₋₆, et
chaque R²³ représente un groupe méthyle, éthyle, propyle ou polyéther.
• de formule (XI) 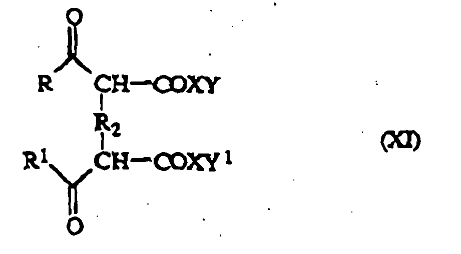 où
R et R¹ représentent chacun un groupe alkyle en C₅₋₃₀, linéaire ou ramifié, saturé ou comportant jusqu' à 2 insaturations non-voisines, éventuellement hydroxylé ou perfluoré,
R² représente un groupe alkylène en C₁₋₁₀ ou arylène éventuellement hydroxylés, un groupe polyéther, -S-, -SO₂-, -O-, -S-S-, -O-R⁵-O- ou -S-R⁵-S-, ou une liaison directe entre les deux atomes de carbone en α,
R⁵ représente un groupe alkylène, arylène ou alkylarylène en C₁₋₁₀, -N(R⁶)-ou-(NR⁶)-R⁷-(NR⁶)-,
R⁶ représente un groupe alkyle en C₁₋₆,
R⁷ représente un groupe alkyle en C₁₋₆,
ou R⁶ et R⁷ forment un hétérocycle,
X représente un groupe polyéther, -O- ou -NZ- avec Z = H, ou alkyle, aryle ou alkylaryle en C₁₋₁₀,
Y et Y¹ représentent chacun indépendamment un atome d'hydrogène ou un groupe -CH₂-COOH éventuellement salifié, un résidu carbohydrate comportant au moins deux groupes hydroxyle,
• de formule (XII) : 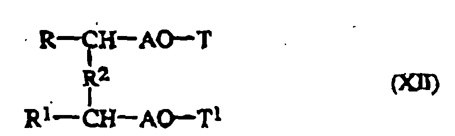 où les symboles ont la signification indiquée pour la formule (XI) et AO représente un groupe -C(O)-, -C(O)-[-O(R⁴O)ₓ], -CH₂-[O(R⁴O)ₓ-)], ou -CH₂-O,
R⁴ représente un groupe alkylène en C₂₋₄,
T et T¹ représentent indépendamment chacun un groupe -OM, -H, -CH₃, -C₂H₅, -SO₃M, -CH₂COOM, -C₂H₄-COOM, -C₃H₆-CO₃M, -O-, P(OXOM)₂ et M représente un ion alcalin ou un demi-ion alcalinoterreux, ou un ion mono-, di- ou trialcanolammonium ou un proton.
• de formule (XIII) 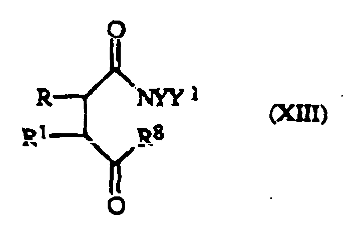 où les symboles ont la signification indiquée pour les formules (XI) et (XII) et R⁸ représente un groupe NYY¹, -O(R⁴O)ₓH ou -O(R⁴O)ₓ-C(O)-CHR-CHR¹-C(O)NYY¹.
• de formule (XIV) 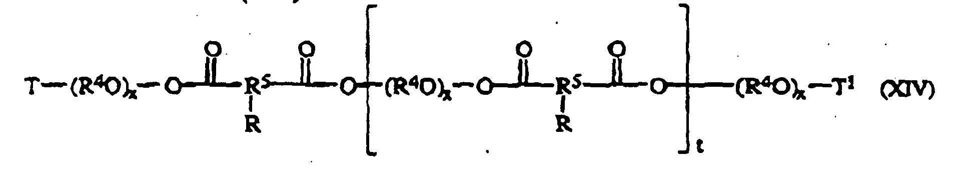 où les symboles ont la signification indiquée pour les formules (XI) à (XIII) et τ représente un nombre entier allant de 1 à 100, de préférence de 1 à 4.

4. Composition photoprotectrice selon l'une quelconque des revendications précédentes, **caractérisée par le fait que** l'agent tensioactif dimère est un agent tensioactif dimère anionique.

5. Composition photoprotectrice selon la revendication 4, **caractérisée par le fait que** l'agent tensioactif dimère est un agent tensioactif de formule (I) : 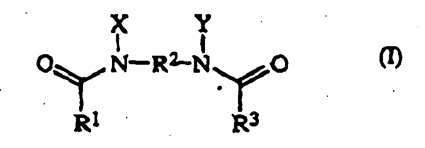 où
R¹ et R³ sont identiques et représentent chacun un groupe alkyle linéaire en C₈₋₁₆, R² représente un groupe alkylène en C₂₋₈. X et Y représentent chacun un groupe (C₂H₄O)ₓ-RF avec x = 10 - 15 et RF = -SO₃M, où M représente un ion d'un métal alcalin.

6. Composition photoprotectrice selon la revendication 5, **caractérisée par le fait que** l'agent tensioactif dimère est le sodium dicocoylethylenediamine PEG-15 sulfate (nom INCI) correspondant à la formule suivante :

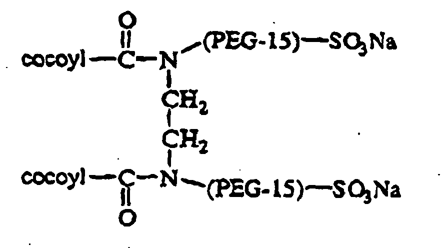

7. Composition photoprotectrice selon l'une quelconque des revendications précédentes, **caractérisée par le fait que** la concentration du ou des agents tensioactifs dimère(s) est comprise entre 0,001 et 8 %, de préférence entre 0,01 et 4 % et en particulier entre 0,05 et 3 % en poids, rapporté au poids total la composition photoprotectrice.

8. Composition photoprotectrice selon l'une quelconque des revendications précédentes, **caractérisée par le fait que** les polymères associatifs non-ioniques sont choisis parmi les celluloses, en particulier les hydroxyéthylcelluloses, modifiées par des groupements comportant au moins une chaîne grasse, les hydroxypropylguars modifiés par des groupements comportant au moins une chaîne grasse, les copolymères de vinylpyrrolidone et de monomères hydrophobes à chaîne grasse, les copolymères de (meth)acrylates d'alkyle en C₁₋₆ et de monomères amphiphiles comportant au moins une chaîne grasse, les copolymères de (méth)acrylates hydrophiles et de monomères hydrophobes comportant au moins une chaîne grasse, les polyéther-polyuréthannes comportant des séquences hydrophiles et au moins une chaîne grasse, et les copolymères de PEG-180, de tétramethoxyméthylglycouril et de laurcht-50 ou d'octoxynol-40.

9. Composition photoprotectrice selon l'une quelconque des revendications précédentes, **caractérisée par le fait que** la concentration du ou des copolylmères associatifs est comprise entre 0,01 et 10 %, de préférence cotre 0,1 et 5 %, rapportée au poids total de la composition photoprotectrice de la présente invention.

10. Composition photoprotectrice selon l'une quelconque des revendications 2 à 9, **caractérisée par le fait que** le ou les filtres UV-A et/ou UV-B organiques sont choisis parmi les dérivés cinnamiques, les dérivés de dibenzoylméthane, les dérivés salicyliques, les dérivés du benzylidènecamphre, les dérivés de triazine, les dérivés de la benzophénonc, les dérivés de β,β'-diphénylacrylate, les dérivés de benzotriazole, les dérivés de benzalmalonate, les 4,4-diarylbutadiènes, les dérivés de bis-benzoxazolyle, les dérivés de méthylène bis-(hydroxyphénylbenzotriazole), les dérivés de phenylbenzimidazole, les dérivés anthraniliques, les dérivés d'imidazolines, les dérivés de l'acide p-aminobenzoïque, les polymères hydrocarbonés filtres et les silicones filtres, les dimères dérivés d'a-alkylstyrène et les mélanges de ces filtres.

11. Composition photoprotectrice selon l'une quelconque des revendications 2 à 10, **caractérisée par le fait que** la concentration des filtres UV-A et/ou UV-B est comprise entre 0,1 et 30 %, de préférence entre 0,5 et 15 % en poids, rapporté au poids total de la composition photoprotectrice

12. Composition photoprotectrice selon l'une quelconque des revendications précédentes, **caractérisée par le fait que** le ou les nanopigments à base d'oxyde métallique sont choisis parmi les oxydes de titane, de fer, de zinc, de zirconium ou de cérium, éventuellement enrobés.

13. Composition photoprotectrice selon la revendication 12, **caractérisée par le fait que** les nanopigments à base d'oxyde métallique sont des nanopigments à base de dioxyde de titane amorphe ou cristallin.

14. Composition photoprotectrice selon l'une quelconque des revendications précédentes, **caractérisée par le fait que** la concentration des nanopigments est comprise entre 0,5 et 10 % en poids, rapporté au poids total de la composition photoprotectrice.

15. Composition photoprotectrice selon l'une quelconque des revendications précédentes, **caractérisée par le fait qu'**elle contient en outre des agents de bronzage et/ou de brunissage artificiels de la peau.

16. Composition photoprotectrice selon l'une quelconque des revendications précédentes, **caractérisée par le fait qu'**elle a une viscosité, mesurée à 25 °C au moyen d'un viscosimètre Brookfield avec une aiguille 7, inférieure à 200 mPa.s, de préférence comprise entre 10 et 180 mPa.s.

17. Procédé de préparation d'une composition photoprotectrice selon l'une quelconque des revendications 1 à 16, comprenant l'homogénéisation, à une température comprise entre 40 et 70°C et à l'aide d'un mixer ou d'un homogénéisateur de type rotor-stator, d'une phase grasse contenant éventuellement un agent tensioactif géminé, éventuellement un ou plusieurs filtres UV organiques et éventuellement un ou plusieurs nanopigment(s), et d'une phase aqueuse contenant éventuellement un agent tensioactif géminé, éventuellement un ou plusieurs filtres UV organiques et éventuellement un ou plusieurs nanopigment(s), étant entendu qu'an moins une de ces phases contient un agent tensioactif géminé, un polymère associatif non-ionique étant incorporé soit dans la phase aqueuse ou la phase grasse avant homogénéisation, soit dans l'émulsion fine obtenue, après refroidissement jusqu'à une température comprise entre 30 et 40°C.

18. Procédé selon la revendication 17, **caractérisé par le fait qu'**un ou plusieurs nanopigments sont incorporés sous forme pulvérulente dans l'émulsion eau-dans-huile, par une deuxième étape d'homogénéisation de la composition.

## Claims

1. Photoprotective composition for protecting the skin and/or the hair against UV radiation, containing
- as liquid phase, an oil-in-water emulsion, emulsified with at least one dimeric surfactant comprising two surfactant units, which may be identical or different, each consisting of a hydrophilic head and a hydrophobic tail and connected to each other, via the hydrophilic heads, by means of a spacer group,
- a photoprotective system capable of screening out UV rays, containing at least one mineral nanopigment based on metal oxide, and
- at least one nonionic associative polymer comprising at least one C₈₋₄₀ fatty chain.

2. Photoprotective composition according to Claim 1, **characterized in that** the photoprotective system capable of screening out UV rays also contains at least one organic UV-A and/or UV-B screening agent.

3. Photoprotective composition according to Claim 1 or 2, **characterized in that** the dimeric surfactant is chosen from those
- of formula (I): 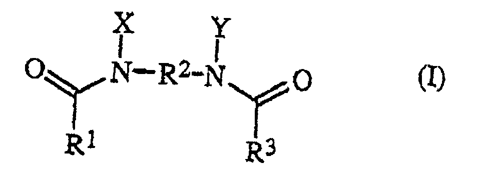 in which
R¹ and R³ represent a linear or branched C₅₋₂₅ alkyl group, which is saturated or containing up to two non-vicinal unsaturations,
R² represents a C₁₋₁₂ alkylene group,
X and Y each represent a group (C₂H₄O)ₓ(C₃H₆O)_{y}-RF with x = 0-15, y = 0-10, x+y ≥ 1, and RF = -SO₃M, -CH₂-CO₂M, -P(O)(OM)₂, H, -C₃H₆SO₃M or a -CH₂(CHOH)₄CH₂OH group when x+y = 0, and
M represents an alkali metal ion, (alkyl)ammonium, alkanolammonium, H or a 1/2 alkaline-earth metal ion,
- of formula (II): 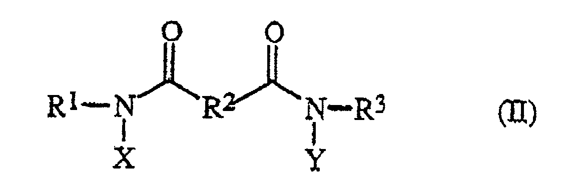 in which the symbols have the same meaning as for formula (I),
- of formula (III), 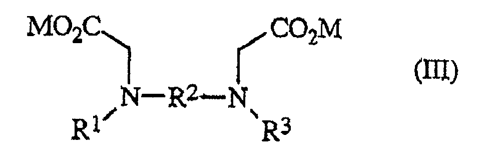 in which the symbols have the meaning indicated for formula (I),
- of formula (IV): 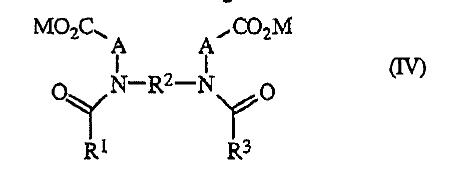 in which
R¹ and R³ represent a linear or branched C₅₋₂₅ alkyl group, which is saturated or containing up to two non-vicinal unsaturations,
R² represents a C₁₋₁₂ alkylene group,
A represents a group -CHR⁴-, -CH₂-, -C₂H₄-, -C₃H₆- or -C₄H₈-,
R⁴ represents an aminocarboxylic acid residue, and
M represents an alkali metal ion, (alkyl)ammonium, alkanolammonium, H or a 1/2 alkaline-earth metal ion,
- of formula (V): 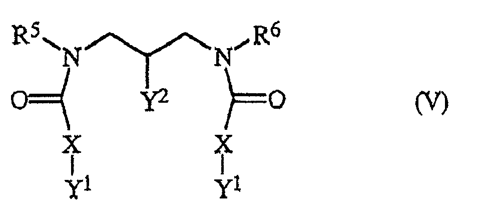 in which
R⁵ and R⁶ represent a linear or branched C₆₋₃₆ alkyl group, which is saturated or containing up to two non-vicinal unsaturations,
X represents an alkylene or alkenylene group containing from 1 to 6 carbon atoms, which can bear a hydroxyl, sulfonic acid or carboxylic acid group,
each Y¹ independently represents a sulfonate, sulfate, carboxyl or hydroxyl group, a sulfuric acid group or -O-(CO)ₓ-COOH,
- of formula (VI): 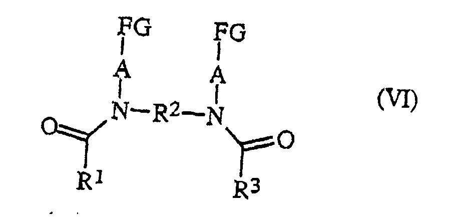 in which the symbols have the meaning given for formula (IV) and FG represents a group -COOM or -SO₃M,
- of formula (VII): 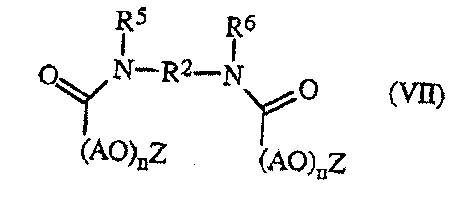 in which the substituents have the meaning indicated for formulae (IV) and (V),
AO represents an alkyleneoxy unit, for example ethyleneoxy, propyleneoxy and butyleneoxy, n = 1 to 20, the alkyleneoxy units possibly being linked together randomly or in blocks, and
Z represents a group -SO₃M, -C₂H₄SO₃M, -C₃H₆SO₃M, -P(O)(OM)₂, -CH₂-COOM or -C₂H₄-COOM,
- of formula (VIII): 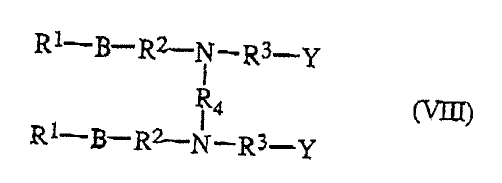 in which
each R¹ represents a linear or branched, optionally hydroxylated or perfluorinated C₅₋₂₅ alkyl group, which is saturated or containing up to two non-vicinal unsaturations,
R² represents an optionally hydroxylated C₁₋₁₂ alkylene group,
B represents an amide, carboxyl or polyether group,
R³ represents an optionally hydroxylated C₁₋₁₂ alkyl group, a group R⁷-D-R⁷ or a polyether group, in which R⁷ represents an optionally hydroxylated C₁₋₆ alkylene group,
D represents a group -O-, -S- or -NR⁸-,
R⁴ represents an optionally hydroxylated alkylene or alkylarylene group comprising from 1 to 12 carbon atoms, or a group R⁹-D¹-R⁹,
R⁸ represents an optionally hydroxylated C₁₋₁₂ alkyl group, a hydrogen atom or a group R⁹-D¹-R⁹,
R⁹ represents an optionally hydroxylated C₁₋₆ alkylene group or an aryl group,
D¹ = -O-, -S-, -SO₂-, -C(O)-, -O(R⁷-O)ₓ-, (R¹⁰)ₜ[N-(R¹⁰)]_{z} or an aryl group,
R¹⁰ represents an optionally substituted C₁₋₁₂ alkyl group, a hydrogen atom or an aryl group,
t and z are each independently a number between 1 and 4, and each Y independently represents an -SO₃H, -OSO₃H, -OP(O)(OH)₂, -P(O)(OH)₂, -COOH or -CO₂-C₆H₄-SO₃H group and the corresponding salts,
- of formula (IX) : 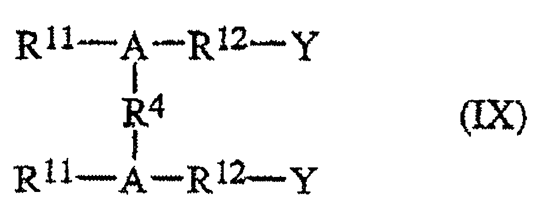
in which each R¹¹ represents a linear or branched, optionally hydroxylated or perfluorinated C₅₋₁₂ alkyl group, which is saturated or comprising up to two non-vicinal unsaturations, or a group R¹⁴-B-R²,
R¹⁴ represents a linear or branched, optionally hydroxylated C₁₋₁₂ alkyl group, which is saturated or comprising up to two non-vicinal unsaturations,
R¹² represents a linear or branched, optionally hydroxylated C₁₋₁₂ alkyl group, which is saturated or comprising up to two non-vicinal unsaturations, or an amide, carboxyl, polyether or R⁹-D¹-R⁹ group, and
A represents a group -CR²= or -N=, with the proviso that, when A represents an -N= group, then R¹¹ is a group R¹⁴-B-R²,
R², R⁴, B, R⁹ and D¹ having the meaning given for formula (VIII),
- of formula (X) : 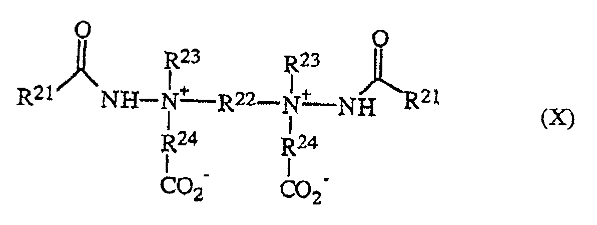 in which
each R²¹ represents a linear or branched C₅₋₂₃ alkyl group, which is saturated or comprising up to 2 non-vicinal unsaturations,
R²² and each R²⁴ represent a C₁₋₆ alkylene group, and
each R²³ represents a methyl, ethyl, propyl or polyether group,
- of formula (XI) : 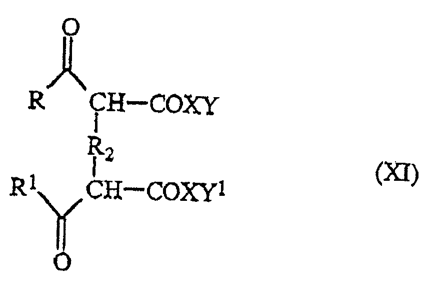 in which
R and R¹ each represent a linear or branched, optionally hydroxylated or perfluorinated C₅₋₃₀ alkyl group, which is saturated or comprising up to 2 non-vicinal unsaturations,
R² represents an optionally hydroxylated C₁₋₁₀ alkylene or arylene group, a polyether group, -S-, -SO₂-, -O-, -S-S-, -O-R⁵-O- or -S-R⁵-S-, or a direct bond between the two α carbon atoms,
R⁵ represents a C₁₋₁₀ alkylene, arylene or alkylarylene group, -N(R⁶)- or -(NR⁶)-R⁷-(NR⁶)-,
R⁶ represents a C₁₋₆ alkyl group,
R⁷ represents a C₁₋₆ alkyl group,
or R⁶ and R⁷ form a heterocycle,
X represents a polyether group, -O- or -NZ- with Z = H or alkyl, aryl or C₁₋₁₀ alkylaryl,
Y and Y¹ each independently represent a hydrogen atom or an optionally salified -CH₂-COOH group, a carbohydrate residue comprising at least two hydroxyl groups,
- of formula (XII): 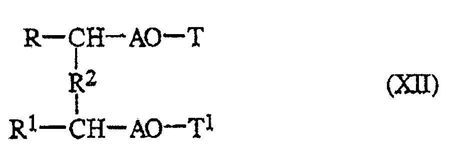 in which the symbols have the meaning indicated for formula (XI) and AO represents a group -C(O)-, -C(O)-[-O(R⁴O)ₓ], -CH₂-[O(R⁴O)ₓ-)] or -CH₂-O,
R⁴ represents a C₂₋₄ alkylene group,
T and T¹ each independently represent a group -OM, -H, -CH₃, -C₂H₅, -SO₃M, -CH₂COOM, -C₂H₄-COOM, -C₃H₆-CO₃M, -O-, P(O)(OM)₂ and M represents an alkali metal ion or an alkaline-earth metal half-ion, or a mono-, di- or trialkanolammonium ion or a proton,
- of formula (XIII): 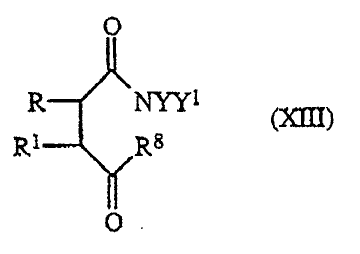 in which the symbols have the meaning indicated for formulae (XI) and (XII) and R⁸ represents a group NYY¹, -O(R⁴O)ₓH or -O(R⁴O)ₓ-C(O)-CHR-CHR¹-C(O)NYY¹,
- of formula (XIV) : 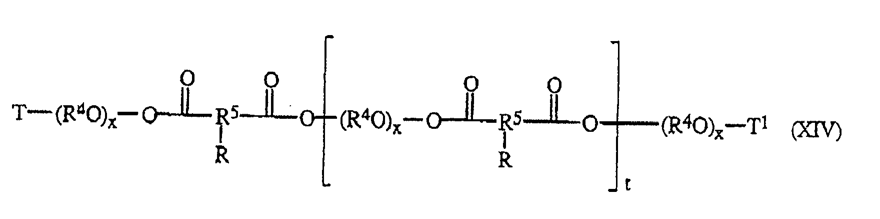 in which the symbols have the meaning indicated for formulae (XI) to (XIII) and t represents an integer ranging from 1 to 100 and preferably from 1 to 4.

4. Photoprotective composition according to any one of the preceding claims, **characterized in that** the dimeric surfactant is an anionic dimeric surfactant.

5. Photoprotective composition according to Claim 4, **characterized in that** the dimeric surfactant is a surfactant of formula (I): 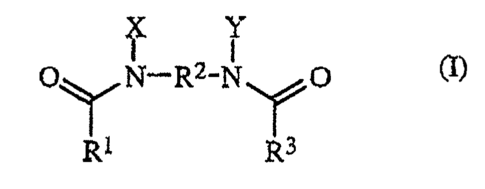 in which
R¹ and R³ are identical and each represent a linear C₈₋₁₆ alkyl group, R² represents a C₂₋₈ alkylene group, X and Y each represent a group (C₂H₄O)ₓ-RF with x = 10-15 and RF = -SO₃M, in which M represents an alkali metal ion.

6. Photoprotective composition according to Claim 5, **characterized in that** the dimeric surfactant is sodium dicocoylethylenediamine PEG-15 sulfate (INCI name) corresponding to the following formula:

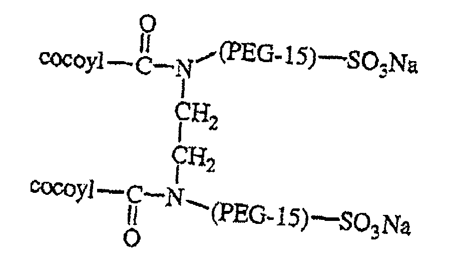

7. Photoprotective composition according to any one of the preceding claims, **characterized in that** the concentration of the dimeric surfactant(s) is between 0.001% and 8%, preferably between 0.01% and 4% and in particular between 0.05% and 3% by weight relative to the total weight of the photoprotective composition.

8. Photoprotective composition according to any one of the preceding claims, **characterized in that** the nonionic associative polymers are chosen from celluloses, in particular hydroxyethylcelluloses, modified with groups comprising at least one fatty chain, hydroxypropyl guars modified with groups comprising at least one fatty chain, copolymers of vinylpyrrolidone and of fatty-chain hydrophobic monomers, copolymers of C₁-C₆ alkyl methacrylates or acrylates and of amphiphilic monomers comprising at least one fatty chain, copolymers of hydrophilic methacrylates or acrylates and of hydrophobic monomers comprising at least one fatty chain, polyether-polyurethanes comprising hydrophilic blocks and at least one fatty chain, and copolymers of PEG-180, of tetramethoxymethylglycouril and of laureth-50 or octoxynol-40.

9. Photoprotective composition according to any one of the preceding claims, **characterized in that** the concentration of the associative copolymer(s) is between 0.01% and 10% and preferably between 0.1% and 5% relative to the total weight of the photoprotective composition of the present invention.

10. Photoprotective composition according to any one of Claims 2 to 9, **characterized in that** the organic UV-A and/or UV-B screening agent(s) is (are) chosen from cinnamic derivatives, dibenzoylmethane derivatives, salicylic derivatives, benzylidenecamphor derivatives, triazine derivatives, benzophenone derivatives, β,β'-diphenylacrylate derivatives, benzotriazole derivatives, benzalmalonate derivatives, 4,4-diarylbutadienes, bis-benzoxazolyl derivatives, methylenebis(hydroxyphenylbenzotriazole) derivatives, phenylbenzimidazole derivatives, anthranilic derivatives, imidazoline derivatives, p-aminobenzoic acid derivatives, screening hydrocarbon-based polymers and screening silicones, α-alkylstyrene-based dimers, and mixtures of these screening agents.

11. Photoprotective composition according to any one of Claims 2 to 10, **characterized in that** the concentration of UV-A and/or UV-B screening agents is between 0.1% and 30% and preferably between 0.5% and 15% by weight relative to the total weight of the photoprotective composition.

12. Photoprotective composition according to any one of the preceding claims, **characterized in that** the metal oxide-based nanopigment(s) is (are) chosen from titanium oxide, iron oxide, zinc oxide, zirconium oxide and cerium oxide, which are optionally coated.

13. Photoprotective composition according to Claim 12, **characterized in that** the metal oxide-based nanopigments are nanopigments based on amorphous or crystalline titanium dioxide.

14. Photoprotective composition according to any one of the preceding claims, **characterized in that** the nanopigment concentration is between 0.5% and 10% by weight relative to the total weight of the photoprotective composition.

15. Photoprotective composition according to any one of the preceding claims, **characterized in that** it also contains agents for artificially tanning and/or browning the skin.

16. Photoprotective composition according to any one of the preceding claims, **characterized in that** it has a viscosity, measured at 25°C using a Brookfield viscometer with a No. 7 needle, of less than 200 mPa.s and preferably between 10 and 180 mPa.s.

17. Process for preparing a photoprotective composition according to any one of Claims 1 to 16, comprising the homogenization, at a temperature of between 40 and 70°C and using a mixer or a homogenizer of rotor-stator type, of a fatty phase optionally containing a gemini surfactant, optionally one or more organic UV-screening agents, and optionally one or more nanopigment(s), and of an aqueous phase optionally containing a gemini surfactant, optionally one or more organic UV-screening agents and optionally one or more nanopigment(s), it being understood that at least one of these phases contains a gemini surfactant, a nonionic associative polymer being incorporated either into the aqueous phase or into the fatty phase before homogenization, or into the fine emulsion obtained, after cooling to a temperature of between 30 and 40°C.

18. Process according to Claim 17, **characterized in that** one or more nanopigments are incorporated in pulverulent form into the water-in-oil emulsion, via a second step of homogenization of the composition.

## Patentansprüche

1. Lichtschutzzusammensetzung, die für den Schutz der Haut und/oder der Haare gegen UV-Strahlung vorgesehen ist und enthält:
- als flüssige Phase eine Öl-in-Wasser-Emulsion, die mit mindestens einem dimeren grenzflächenaktiven Stoff emulgiert ist, der zwei identische oder voneinander verschiedene grenzflächenaktive Einheiten aufweist, die jeweils aus einem hydrophilen Kopf und einem hydrophoben Schwanz bestehen und die auf der Höhe der hydrophilen Köpfe über eine Spacergruppe miteinander verbunden sind,
- ein Lichtschutzsystem, das befähigt ist, UV-Strahlung zu filtern, und das mindestens ein anorganisches Nanopigment auf der Basis eines Metalloxids enthält, und
- mindestens ein nichtionisches assoziatives Polymer, das mindestens eine Fettkette mit 8 bis 40 Kohlenstoffatomen aufweist.

2. Lichtschutzzusammensetzung nach Anspruch 1, **dadurch gekennzeichnet, dass** das Lichtschutzsystem, das befähigt ist, UV-Strahlung zu filtern, ferner mindestens ein organisches UV-A- und/oder UV-B-Filter enthält.

3. Lichtschutzzusammensetzung nach Anspruch 1 oder 2, **dadurch gekennzeichnet, dass** der dimere grenzflächenaktive Stoff ausgewählt ist unter den Verbindungen
• der Formel (I) 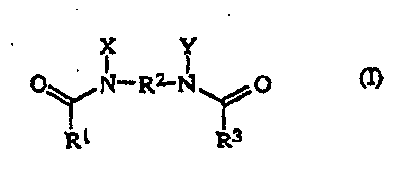
wobei R¹ und R³ eine geradkettige oder verzweigte C₅₋₂₅-Alkylgruppe bedeuten, die gesättigt ist oder bis zu zwei ungesättigte Bindungen enthält, die nicht benachbart sind,
R² eine C₁₋₁₂-Alkylengruppe bedeutet,
X und Y jeweils eine Gruppe (C₂H₄O)ₓ(C₃H₆O)_{y}-RF bedeuten, mit x = 0 - 15, y = 0 - 10, x + y ≥ 1 und RF = -SO₃M, -CH₂-CO₂M, -P(O)(OM)₂, H, -C₃H₆SO₃M, oder eine Gruppe -CH₂(CHOH)₄CH₂OH, wenn x + y = 0, und
M ein Alkaliion, Alkyl(ammonium), Alkanolammonium, H oder 1/2 Erdalkaliion bedeutet;
• der Formel (II) 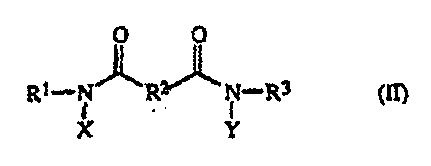 wobei die Symbole die für die Formel (I) angegebenen Bedeutungen aufweisen,
• der Formel (III), 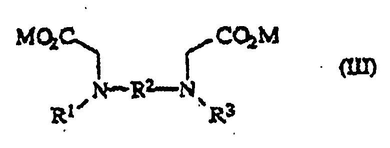 wobei die Symbole die für die Formel (I) angegebenen Bedeutungen aufweisen,
• der Formel (IV) 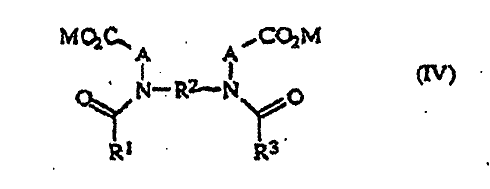
wobei R¹ und R³ eine geradkettige oder verzweigte C₅₋₂₅-Alkylgruppe bedeuten, die gesättigt ist oder bis zu zwei ungesättigte Bindungen enthält, die nicht benachbart sind,
R² eine C₁₋₁₂-Alkylengruppe,
A eine Gruppe -CHR⁴-, -CH₂-, -C₂H₄-, -C₃H₆-, -C₄H₈-,
R⁴ den Rest einer Aminocarbonsäure und
M ein Alkaliion, (Alkyl)ammonium, Alkanolammonium, H oder 1/2 Erdalkaliion,
• der Formel (V) 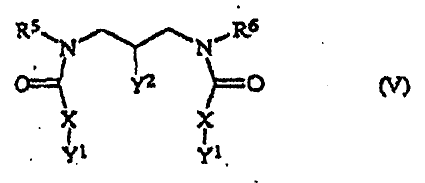
wobei R⁵ und R⁶ eine geradkettige oder verzweigte C₆₋₃₆-Alkylgruppe bedeuten, die gesättigt ist oder bis zu zwei ungesättigte Bindungen enthält, die nicht benachbart sind,
X eine Alkylen- oder Alkenylengruppe ist, die 1 bis 6 Kohlenstoffatome enthält und eine Hydroxygruppe, Sulfonsäuregruppe oder Carbonsäuregruppe tragen kann,
jede Gruppe Y' unabhängig eine Gruppe Sulfonat, Sulfat, Carboxy oder Hydroxy, eine Schwefelsäuregruppe oder -O-(CO)ₓ-COOH bedeutet,
• der Formel (VI) 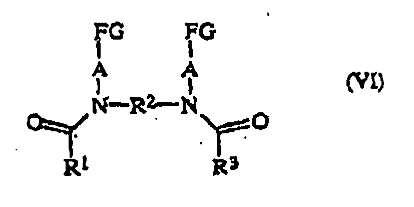 wobei die Symbole die für die Formel (IV) angegebenen Bedeutungen aufweisen und FG eine Gruppe -COOM oder -SO₃M bedeutet,
• der Formel (VII) 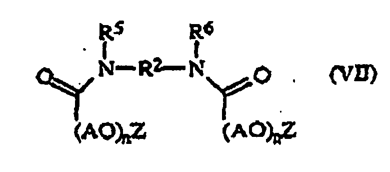
wobei die Substituenten die für die Formeln (IV) und (V) angegebenen Bedeutungen aufweisen,
AO eine Alkylenoxyeinheit bedeutet, beispielsweise Ethylenoxy, Propylenoxy und Butylenoxy, n = 1 bis 20, wobei die Alkylenoxyeinheiten statistisch oder blockweise verknüpft sein können, und
Z eine Gruppe -SO₃M, -C₂H₄SO₃M, -C₃H₆SO₃M, -P(O)(OM)₂, -CH₂-COOM oder -C₂H₄-COOM bedeutet,
• der Formel (VIII) 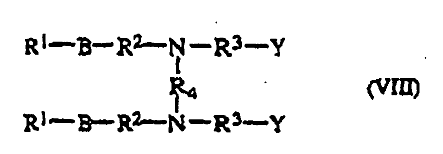
wobei jede Gruppe R¹ eine geradkettige oder verzweigte C₅₋₂₅-Alkylgruppe bedeutet, die gesättigt ist oder bis zu zwei ungesättigte Bindungen enthalten kann, die nicht benachbart sind, und gegebenenfalls hydroxyliert oder perfluoriert ist,
R² eine C₁₋₁₂-Alkylengruppe bedeutet, die gegebenenfalls hydroxyliert ist,
B eine Gruppe Amid, Carboxy oder Polyether bedeutet,
R³ eine C₁₋₁₂-Alkylgruppe, die gegebenenfalls hydroxyliert ist, eine Gruppe R⁷-D-R⁷ oder eine Polyethergruppe bedeutet, wobei R⁷ eine C₁₋₆-Alkylengruppe ist, die gegebenenfalls hydroxyliert ist,
D eine Gruppe -O-, -S- oder -NR₈- ist,
R⁴ eine Alkylen- oder Alkylarylengruppe, die 1 bis 12 Kohlenstoffatome aufweist und gegebenenfalls hydroxyliert ist, oder eine Gruppe R⁹-D¹-R⁹ bedeutet,
R⁸ eine C₁₋₁₂-Alkylengruppe, die gegebenenfalls hydroxyliert ist, ein Wasserstoffatom oder eine Gruppe R⁹-D¹-R⁹ bedeutet, R⁹ eine C₁₋₆-Alkylengruppe, die gegebenenfalls hydroxyliert ist, oder eine Arylgruppe ist,
D¹ = -O-, -S-, -SO₂-, -C(O)-, -O(R⁷-O)ₓ-, (R¹⁰)ₜ[N(R¹⁰)]_{z} oder eine Arylgruppe,
R¹⁰ eine C₁₋₁₂-Alkylgruppe, die gegebenenfalls substituiert ist, oder ein Wasserstoffatom oder eine Arylgruppe bedeutet,
t und z unabhängig jeweils eine Zahl von 1 bis 4 bedeuten, und jede Gruppe Y unabhängig eine Gruppe -SO₃H, -OSO₃H, -OP(O)(OH)₂, -P(O)(OH)₂, -COOH, -CO₂-C₆H₄-SO₃H und die entsprechenden Salze bedeutet,
• der Formel (IX) 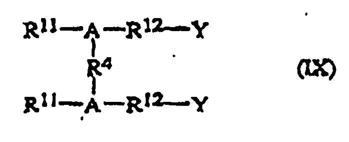
wobei jede Gruppe R¹¹ eine geradkettige oder verzweigte C₅₋₁₂-Alkylgruppe, die gesättigt ist oder bis zu zwei ungesättigte Bindungen aufweist, die nicht benachbart sind, und gegebenenfalls hydroxyliert oder perfluoriert ist, oder eine Gruppe R¹⁴-B-R² bedeutet,
R¹⁴ eine geradkettige oder verzweigte C₁₋₁₂-Alkylgruppe bedeutet, die gesättigt ist oder bis zu zwei ungesättigte Bindungen enthält, die nicht benachbart sind, und gegebenenfalls hydroxyliert ist,
R¹² eine geradkettige oder verzweigte C₁₋₁₂-Alkylgruppe bedeutet, die gesättigt ist oder bis zu zwei ungesättigte Bindungen aufweist, die nicht benachbart sind, und gegebenenfalls hydroxyliert ist, oder eine Gruppe Amid, Carboxy, Polyether oder R⁹-D¹-R⁹, und
A eine Gruppe -CR²= oder -N= bedeutet, mit der Maßgabe, dass R¹¹ eine Gruppe R¹⁴-B-R² ist, wenn A eine Gruppe -N= bedeutet, wobei
R², R⁴, B, R⁹ und D¹ die oben für die Formel (VIII) angegebenen Bedeutungen aufweisen,
• der Formel (X) 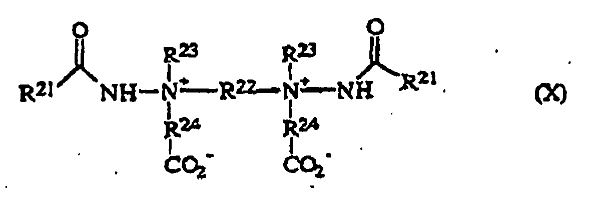
wobei jede Gruppe R²¹ eine geradkettige oder verzweigte C₅₋₂₃-Alkylgruppe bedeutet, die gesättigt ist oder bis zu 2 ungesättigte Bindungen aufweist, die nicht benachbart sind,
R22 und jede Gruppe R²⁴ eine C₁₋₆-Alkylengruppe bedeuten, und
jede Gruppe R²³ eine Gruppe Methyl, Ethyl, Propyl oder Polyether bedeutet,
• der Formel (XI) 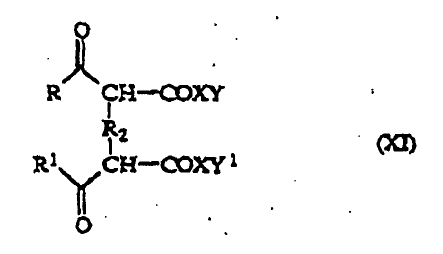
wobei R und R¹ jeweils eine geradkettige oder verzweigte C₅₋₃₀-Alkylgruppe bedeuten, die gesättigt ist oder bis zu zwei ungesättigte Bindungen aufweist, die nicht benachbart sind, und gegebenenfalls hydroxyliert oder perfluoriert ist,
R² eine C₁₋₁₀-Alkylengruppe oder eine Arylengruppe, die gegebenenfalls hydroxyliert sind, eine Polyethergruppe, -S-, -SO₂-, -O-, -S-S-, -O-R⁵-O- oder -S-R⁵-S- oder eine direkte Bindung zwischen den beiden Kohlenstoffatomen in α-Stellung bedeutet,
R⁵ eine Alkylen-, Arylen- oder Alkylarylengruppe mit 1 bis 10 Kohlenstoffatomen, -N(R⁶)- oder -(NR⁶)-R⁷-(NR⁶)- bedeutet,
R⁶ eine C₁₋₆-Alkylgruppe ist,
R⁷ eine C₁₋₆-Alkylgruppe ist,
oder R⁶ und R⁷ einen Heterocyclus bilden,
X eine Polyethergruppe, -O- oder -NZ- mit Z = H oder Alkyl,
Aryl oder Alkylaryl mit 1 bis 10 Kohlenstoffatomen bedeutet, Y und Y' jeweils unabhängig ein Wasserstoffatom oder eine Gruppe -CH₂-COOH, die gegebenenfalls in Salzform vorliegt, einen Kohlenhydratrest bedeuten, der mindestens zwei Hydroxygruppen aufweist,
• der Formel (XII) 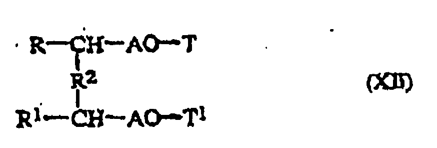
wobei die Symbole die für die Formel (XI) angegebenen Bedeutungen aufweisen und AO eine Gruppe -C(O)-, -C(O)-[-O(R⁴O)ₓ], -CH₂-[O(R⁴O)ₓ-)] oder -CH₂O bedeutet,
R⁴ eine C₂₋₄-Alkylengruppe ist,
T und T¹ unabhängig jeweils eine Gruppe -OM, -H, -CH₃, -C₂H₅, -SO₃M, -CH₂COOM, -C₂H₄-COOM, C₃H₆-CO₃M, -O-, P(O)(OM)₂ bedeuten, und
M ein Alkaliion oder ein halbes Erdalkaliion, ein Mono-, Di- oder Trialkanolammoniumion oder ein Proton bedeutet;
• der Formel (XIII) 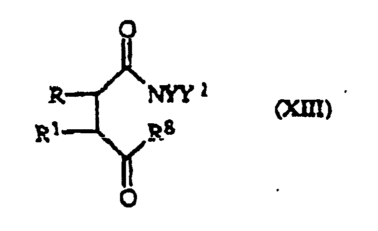 wobei die Symbole die für die Formeln (XI) und (XII) angegebenen Bedeutungen aufweisen und R⁸ eine Gruppe NYY¹, -O(R⁴O)ₓH oder -O(R⁴O)ₓ-C(O)-CHR-CHR¹-C(O)NYY¹ bedeutet;
• der Formel (XIV) 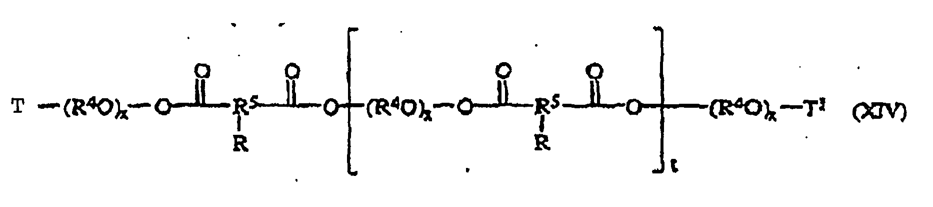 wobei die Symbole die für die Formeln (XI) bis (XIII) angegebenen Bedeutungen aufweisen und t eine ganze Zahl im Bereich von 1 bis 100 und vorzugsweise 1 bis 4 ist.

4. Lichtschutzzusammensetzung nach einem der vorhergehenden Ansprüche, **dadurch gekennzeichnet, dass** der dimere grenzflächenaktive Stoff ein anionischer dimerer grenzflächenaktiver Stoff ist.

5. Lichtschutzzusammensetzung nach Anspruch 4, **dadurch gekennzeichnet, dass** der dimere grenzflächenaktive Stoff ein grenzflächenaktiver Stoff der Formel (I) ist: 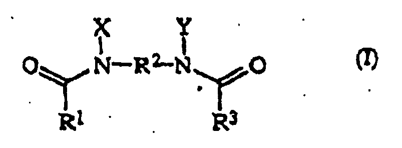 wobei
die Gruppe R¹ und R³ identisch sind und jede eine lineare Alkylgruppe mit 8 bis 16 Kohlenstoffatomen bedeuten, R² eine Alkylengruppe mit 2 bis 8 Kohlenstoffatomen ist, X und Y jeweils eine Gruppe (C₂H₄O)ₓ-RF mit x = 10 - 15 und RF = -SO₃M bedeuten, wobei M ein Alkalimetallion ist.

6. Lichtschutzzusammensetzung nach Anspruch 5, **dadurch gekennzeichnet, dass** der dimere grenzflächenaktive Stoff das Sodium Dicocoylethylenediamine PEG-15 Sulfate (INCI-Name) der folgenden Formel ist:

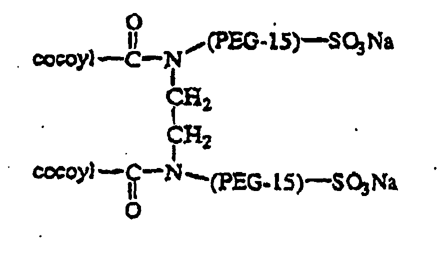

7. Lichtschutzzusammensetzung nach einem der vorhergehenden Ansprüche, **dadurch gekennzeichnet, dass** die Konzentration des oder der dimeren grenzflächenaktiven Stoffe(s) im Bereich von 0,001 bis 8 Gew.-%, vorzugsweise 0,01 bis 4 Gew.-% und insbesondere 0,05 bis 3 Gew.-%, bezogen auf das Gesamtgewicht der Lichtschutzzusammensetzung, liegt.

8. Lichtschutzzusammensetzung nach einem der vorhergehenden Ansprüche, **dadurch gekennzeichnet, dass** die nichtionischen assoziativen Polymere unter den Cellulosen, insbesondere Hydroxyethylcellulosen, die mit Gruppen modifiziert sind, die mindestens eine Fettkette aufweisen, Hydroxypropylguarverbindungen, die mit Gruppen modifiziert sind, die mindestens eine Fettkette aufweisen, Copolymeren von Vinylpyrrolidon und hydrophoben Monomeren mit Fettkette, Copolymeren von Alkyl(C₁₋₆)(meth)acrylaten und amphiphilen Monomeren, die mindestens eine Fettkette aufweisen, Copolymeren von hydrophilen (Meth)acrylaten und hydrophoben Monomeren, die mindestens eine Fettkette aufweisen, Polyetherpolyurethanen, die hydrophile Sequenzen und mindestens eine Fettkette aufweisen, und Copolymeren von PEG-180, Tetramethoxymethylglycouril und Laureth-50 oder Octoxynol-40 ausgewählt sind.

9. Lichtschutzzusammensetzung nach einem der vorhergehenden Ansprüche, **dadurch gekennzeichnet, dass** die Konzentration des oder der assoziativen Copolymere(s) im Bereich von 0,01 bis 10 % und vorzugsweise 0,1 bis 5 %, bezogen auf das Gesamtgewicht der Lichtschutzzusammensetzung der vorliegenden Erfindung, liegt.

10. Lichtschutzzusammensetzung nach einem der Ansprüche 2 bis 9, **dadurch gekennzeichnet, dass** das oder die organischen UV-A- und/ oder UV-B-Filter unter den Zimtsäurederivaten, Dibenzoylmethanderivaten, Salicylsäurederivaten, Benzylidencampherderivaten, Triazinderivaten, Benzophenonderivaten, β,β-Diphenylacrylatderivaten, Benzotriazolderivaten, Benzalmalonatderivaten, 4,4-Diarylbutadienen, Bis-benzoxazolylderivaten, Methylen-bis(hydroxyphenylbenzotriazolderivaten), Phenylbenzimidazolderivaten, Anthranilderivaten, Imidazolinderivaten, p-Aminobenzoesäurederivaten, Filtern auf Basis von Kohlenwasserstoffpolymeren und Siliconfiltern, von α-Alkylstyrol abgeleiteten Dimeren und Gemischen dieser Filter ausgewählt sind.

11. Lichtschutzzusammensetzung nach einem der Ansprüche 2 bis 10, **dadurch gekennzeichnet, dass** die Konzentration der UV-A- und/oder UV-B-Filter im Bereich von 0,1 bis 30 % und vorzugsweise 0,5 bis 15 Gew.-%, bezogen auf das Gesamtgewicht der Lichtschutzzusammensetzung, liegt.

12. Lichtschutzzusammensetzung nach einem der vorhergehenden Ansprüche, **dadurch gekennzeichnet, dass** das oder die Nanopigmente auf Basis eines Metalloxids unter den Oxiden von Titan, Eisen, Zink, Zirconium oder Cer, die gegebenenfalls umhüllt sind, ausgewählt sind.

13. Lichtschutzzusammensetzung nach Anspruch 12, **dadurch gekennzeichnet, dass** die Nanopigmente auf der Basis eines Metalloxids Nanopigmente auf der Basis von amorphem oder kristallinen Titandioxid sind.

14. Lichtschutzzusammensetzung nach einem der vorhergehenden Ansprüche, **dadurch gekennzeichnet, dass** die Konzentration der Nanopigmente im Bereich von 0,5 bis 10 Gew.-%, bezogen auf das Gesamtgewicht der Lichtschutzzusammensetzung, liegt.

15. Lichtschutzzusammensetzung nach einem der vorhergehenden Ansprüche, **dadurch gekennzeichnet, dass** sie ferner Bräunungsmittel und/oder Mittel zur künstlichen Braunfärbung der Haut enthält.

16. Lichtschutzzusammensetzung nach einem der vorhergehenden Ansprüche, **dadurch gekennzeichnet, dass** sie eine bei 25 °C mit einem Brookfield-Viskosimeter mit Nadel 7 gemessene Viskosität unter 200 mPa.s und vorzugsweise im Bereich von 10 bis 180 mPa·s aufweist.

17. Verfahren zur Herstellung einer Lichtschutzzusammensetzung nach einem der Ansprüche 1 bis 16, das die Homogenisierung einer Fettphase, die gegebenenfalls ein Gemini-Tensid, gegebenenfalls ein oder mehrere organische UV-Filter und gegebenenfalls ein oder mehrere Nanopigment(e) enthält, und einer wässrigen Phase, die gegebenenfalls ein Gemini-Tensid, gegebenenfalls ein oder mehrere organische UV-Filter und gegebenenfalls ein oder mehrere Nanopigment(e) enthält, bei einer Temperatur im Bereich von 40 bis 70 °C und mit Hilfe eines Mixers oder eines Homogenisators vom Rotor/ Stator-Typ umfasst, mit der Maßgabe, dass mindestens eine dieser Phasen ein Gemini-Tensid enthält, wobei ein nichtionisches assoziatives Polymer entweder vor der Homogenisierung in die wässrige Phase oder in die Fettphase oder nach dem Abkühlen auf eine Temperatur von 30 bis 40 °C in die fertige Emulsion eingearbeitet wird.

18. Verfahren nach Anspruch 17, **dadurch gekennzeichnet, dass** ein oder mehrere Nanopigmente in Pulverform über einen zweiten Schritt der Homogenisierung der Zusammensetzung in die Wasser-in-Öl-Emulsion eingebracht werden.
